# EUROPEAN PATENT APPLICATION

(11) **EP 4 343 327 A1**
(43) Date of publication of application: **27.03.2024**
(21) Application number: 23199030.0
(22) Date of filing: 22.09.2023
(51) Int. Cl.: G01N 33/543, G01N 33/573

(54) **METHOD FOR MEASURING ACTIVITY OF ALKALINE PHOSPHATASE CONTAINED IN EXTRACELLULAR VESICLE, CALIBRATOR, AND CONJUGATE**

(30) Priority: 22.09.2022 JP 2022151929
(71) Applicant: SYSMEX CORPORATION, Kobe-shi Hyogo 651-0073 (JP)
(72) Inventor: TAMADA, Eiya, Kobe-shi, Hyogo, 651-0073 (JP); YANAGIDA, Masatoshi, Kobe-shi, Hyogo, 651-0073 (JP); YAMASHITA, Kazuto, Kobe-shi, Hyogo, 651-0073 (JP); NAKHAEI, Elnaz, Kobe-shi, Hyogo, 651-0073 (JP); ONISHI, Mami, Kobe-shi, Hyogo, 651-0073 (JP); KIRIYAMA, Maria, Kobe-shi, Hyogo, 651-0073 (JP)
(74) Representative: De Clercq & Partners

(57) **Abstract**

Disclosed is a method for measuring an activity of alkaline phosphatase contained in an extracellular vesicle in a sample, including acquiring an activity value of alkaline phosphatase contained in an extracellular vesicle in a sample using a calibrator, in which the calibrator contains a conjugate in which a polypeptide having alkaline phosphatase activity and a polypeptide including an amino acid sequence of a membrane protein present on a surface of the extracellular vesicle are covalently bound.

## Description

### FIELD OF THE INVENTION

The present invention relates to a method for measuring an activity of alkaline phosphatase (ALP) contained in an extracellular vesicle. The present invention relates to a calibrator used for measuring an activity of ALP contained in an extracellular vesicle. The present invention relates to a conjugate suitable for measuring an activity of ALP contained in an extracellular vesicle.

### BACKGROUND

An extracellular vesicle is a nano-sized (tens of nm to hundreds of nm) membrane vesicle surrounded by a lipid bilayer. Extracellular vesicles are secreted from almost all cells. Extracellular vesicles are present in various biological samples such as blood and urine. An extracellular vesicle contains various substances such as proteins, DNA, mRNA, miRNA, lipids, sugar chains, and metabolites derived from cells. In recent years, it has been reported that substances contained in an extracellular vesicle function as an intercellular information transfer molecule and are involved in various physiological or pathological processes. For example, it has been reported that an extracellular vesicle is involved in the calcification of blood vessels (U.S. Patent Application Publication No. 2013/0017562).

Calcification in vivo occurs mainly by deposition of hydroxyapatite on collagen fibers. Since calcification occurring in blood vessels causes ischemic heart disease, cerebrovascular disorder, heart failure, and the like, it is important to construct an analysis system for discriminating calcification of blood vessels. A method for discriminating calcification of blood vessels is described, for example, in U.S. Patent Application Publication No. 2013/0017562, wherein extracellular vesicles in blood are isolated, and a vesicle compound (calcium salt) in the extracellular vesicles is measured. In U.S. Patent Application Publication No. 2013/0017562, extracellular vesicles are broken down, and components contained in the extracellular vesicles are analyzed.

### SUMMARY OF THE INVENTION

As an analysis system for discriminating calcification of blood vessels, the inventors have tried to measure the activity of ALP present on the surface of an extracellular vesicle. However, when the activity of ALP present on the surface of an extracellular vesicle is measured, there is no calibrator that can be used as a control, and the measurement has been difficult. An object of the present invention is to provide a calibrator suitable for measurement of extracellular vesicle and a measurement method using the same.

The present invention provides the inventions of the following items 1 to 18.

### [Item 1]

A method for measuring an activity of alkaline phosphatase contained in an extracellular vesicle in a sample, including
acquiring an activity value of alkaline phosphatase contained in an extracellular vesicle in a sample using a calibrator,
in which the calibrator contains a conjugate in which a polypeptide having alkaline phosphatase activity and a polypeptide comprising an amino acid sequence of a membrane protein present on a surface of the extracellular vesicle are covalently bound.

### [Item 2]

The method according to item 1, including:
a first immobilization step of immobilizing the extracellular vesicle contained in the sample on a first solid phase;
a first measurement step of measuring a signal generated by contacting the immobilized extracellular vesicle with a substrate of alkaline phosphatase;
a second immobilization step of immobilizing the conjugate contained in the calibrator on a second solid phase;
a second measurement step of measuring a signal generated by contacting the immobilized conjugate with a substrate of alkaline phosphatase; and
acquiring the activity value of alkaline phosphatase contained in the extracellular vesicle in the sample from a measured value obtained in the first measurement step and a measured value obtained in the second measurement step.

### [Item 3]

The method according to item 2, in which the first immobilization step comprises contacting the sample, the first solid phase, and a capture antibody capable of binding to the membrane protein contained in the extracellular vesicle in the sample with each other to form an immune complex of the extracellular vesicle and the capture antibody on the first solid phase.

### [Item 4]

The method according to item 2 or 3, in which the second immobilization step comprises contacting the calibrator, the second solid phase, and a capture antibody capable of binding to the membrane protein contained in the conjugate with each other to form an immune complex of the conjugate and the capture antibody on the second solid phase.

### [Item 5]

The method according to any one of items 1 to 4, in which the amino acid sequence of the membrane protein comprises an amino acid sequence of a membrane protein selected from the group consisting of an extracellular domain of CD9, an extracellular domain of CD63, an extracellular domain of CD81, Annexin I, Annexin VI, and Pit 1.

### [Item 6]

The method according to any one of items 1 to 5, in which the alkaline phosphatase is one selected from the group consisting of tissue non-specific alkaline phosphatase, small intestine alkaline phosphatase, placenta alkaline phosphatase, and germ cell alkaline phosphatase.

### [Item 7]

The method according to item 3, in which the capture antibody in the first immobilization step is at least one selected from the group consisting of an anti-CD9 antibody, an anti-CD63 antibody, an anti-CD81 antibody, an anti-Annexin I antibody, an anti-Annexin VI antibody, and an anti-Pit 1 antibody.

### [Item 8]

The method according to item 4, in which the capture antibody in the second immobilization step is at least one selected from the group consisting of an anti-CD9 antibody, an anti-CD63 antibody, an anti-CD81 antibody, an anti-Annexin I antibody, an anti-Annexin VI antibody, and an anti-Pit 1 antibody.

### [Item 9]

The method according to any one of items 1 to 8, in which the polypeptide having alkaline phosphatase activity is a recombinant polypeptide, and/or the polypeptide comprising an amino acid sequence of the membrane protein is a recombinant polypeptide.

### [Item 10]

The method according to any one of items 1 to 9, in which the sample is a blood sample.

### [Item 11]

The method according to any one of items 1 to 10, in which the calibrator is a reagent containing the conjugate.

### [Item 12]

The method according to any one of items 1 to 11, in which the calibrator is a reagent set including a plurality of reagents each containing the conjugate, and the concentrations of the conjugate in the plurality of reagents are different from each other.

### [Item 13]

The method according to item 2, in which
the calibrator is a reagent set comprising a plurality of reagents each comprising the conjugate, and the concentrations of the conjugate in the plurality of reagents are different from each other, and
the second measurement step is performed for each of conjugates derived from a plurality of reagents having different concentrations, and acquiring the activity value of alkaline phosphatase contained in the extracellular vesicle in the sample is performed based on a calibration curve prepared from measured values obtained from the conjugates derived from a plurality of reagents having different concentrations.

### [Item 14]

A calibrator suitable for measuring activity of alkaline phosphatase contained in an extracellular vesicle in a sample, in which
the calibrator contains a conjugate in which a polypeptide having alkaline phosphatase activity and a polypeptide including an amino acid sequence of a membrane protein present on a surface of an extracellular vesicle are covalently bound. And the use of said calibrator in a method for measuring activity of alkaline phosphatase contained in an extracellular vesicle in a sample.

### [Item 15]

The calibrator according to item 14, in which the amino acid sequence of a membrane protein includes an amino acid sequence of a membrane protein selected from the group consisting of an extracellular domain of CD9, an extracellular domain of CD63, an extracellular domain of CD81, Annexin I, Annexin VI, and Pit 1.

### [Item 16]

The calibrator according to item 14 or 15, in which the alkaline phosphatase includes at least one amino acid sequence of an alkaline phosphatase selected from the group consisting of tissue non-specific alkaline phosphatase, small intestine alkaline phosphatase, placenta alkaline phosphatase, and germ cell alkaline phosphatase.

### [Item 17]

The calibrator according to any one of items 14 to 16, in which the polypeptide having alkaline phosphatase activity and/or the polypeptide including an amino acid sequence of a membrane protein is a recombinant polypeptide.

### [Item 18]

A conjugate suitable for measuring activity of alkaline phosphatase contained in an extracellular vesicle in a sample, in which a polypeptide having alkaline phosphatase activity and a polypeptide including an amino acid sequence of a membrane protein present on a surface of an extracellular vesicle are covalently bound. And the use of said conjugate in a method for measuring activity of alkaline phosphatase contained in an extracellular vesicle in a sample

According to the present invention, a calibrator used for measuring an activity of ALP contained in an extracellular vesicle, a measurement method using the same, and a conjugate contained in the calibrator are provided.

### BRIEF DESCRIPTION OF THE DRAWINGS

Fig. 1 is a schematic diagram of a configuration of a conjugate in which a polypeptide having ALP activity and a polypeptide including an amino acid sequence of a membrane protein present on a surface of an extracellular vesicle are covalently bound according to an embodiment of the invention.
Fig. 2 is a schematic diagram of a kit including a calibrator according to an embodiment of the invention.
Fig. 3A is a schematic diagram of a kit including a calibrator according to an embodiment of the invention.
Fig. 3B is a schematic diagram of a kit including a calibrator according to an embodiment of the invention.
Fig. 4 is a block diagram showing a configuration of an immunoassay device 1 according to an embodiment of the invention.
Fig. 5 is a flowchart showing an example of a measurement method using the immunoassay device 1 according to an embodiment of the invention.
Fig. 6 is a graph showing a result of fractionating a conjugate of an extracellular domain of CD9 and ALP (hereinafter, also referred to as a "CD9(ECD)-ALP conjugate", "ECD" represents an extracellular domain) by gel filtration chromatography.
Fig. 7 is a graph showing ALP activity of a CD9(ECD)-ALP conjugate according to an embodiment of the invention.
Fig. 8 is a calibration curve for protein concentration prepared using a calibrator containing a CD9(ECD)-ALP conjugate according to an embodiment of the invention.
Fig. 9 is a calibration curve for an ALP activity value prepared using a calibrator containing a CD9(ECD)-ALP conjugate according to an embodiment of the invention.
Fig. 10 is a graph showing protein concentrations of extracellular vesicles containing ALP in plasma specimen samples of patients with history of cardiovascular disease.
Fig. 11 is a graph showing ALP activity values of extracellular vesicles containing ALP in plasma specimen samples of patients with history of cardiovascular disease.
Fig. 12 is a graph showing protein concentrations of extracellular vesicles containing ALP in each of plasma specimen samples of patients with history of cardiovascular disease and healthy persons.
Fig. 13 is a graph showing a correlation between values of protein concentrations by an automatic measurement method and measured values of ELISA by a manual method.
Fig. 14 is a graph showing ALP activity of a conjugate of full-length CD9 and ALP (hereinafter, also referred to as a "CD9(FL)-ALP conjugate", "FL" represents a full length of the amino acid sequence).
Fig. 15 is a calibration curve for protein concentration prepared using a calibrator containing a CD9(FL)-ALP conjugate according to an embodiment of the invention.
Fig. 16 is a calibration curve for an ALP activity value prepared using a calibrator containing a CD9(FL)-ALP conjugate according to an embodiment of the invention.
Fig. 17 is a graph showing protein concentrations of extracellular vesicles containing ALP in plasma specimen samples of patients with history of cardiovascular disease.
Fig. 18 is a graph showing ALP activity values of extracellular vesicles containing ALP in plasma specimen samples of patients with history of cardiovascular disease.
Fig. 19 is a graph showing ALP activity of a conjugate of an extracellular domain of CD63 and ALP (hereinafter, also referred to as a "CD63(ECD)-ALP conjugate").
Fig. 20 is a calibration curve for protein concentration prepared using a calibrator containing a CD63(ECD)-ALP conjugate according to an embodiment of the invention.
Fig. 21 is a calibration curve for an ALP activity value prepared using a calibrator containing a CD63(ECD)-ALP conjugate according to an embodiment of the invention.
Fig. 22 is a graph showing protein concentrations of extracellular vesicles containing ALP in plasma specimen samples of patients with end-stage renal failure.
Fig. 23 is a graph showing ALP activity values of extracellular vesicles containing ALP in plasma specimen samples of patients with end-stage renal failure.
Fig. 24 is a graph showing ALP activity of a conjugate of an extracellular domain of CD81 and ALP (hereinafter, also referred to as a "CD81(ECD)-ALP conjugate").
Fig. 25 is a calibration curve for protein concentration prepared using a calibrator containing a CD81(ECD)-ALP conjugate according to an embodiment of the invention.
Fig. 26 is a calibration curve for an ALP activity value prepared using a calibrator containing a CD81(ECD)-ALP conjugate according to an embodiment of the invention.
Fig. 27 is a graph showing protein concentrations of extracellular vesicles containing ALP in plasma specimen samples of patients with end-stage renal failure.
Fig. 28 is a graph showing ALP activity values of extracellular vesicles containing ALP in plasma specimen samples of patients with end-stage renal failure.
Fig. 29 is a graph showing ALP activity of a conjugate of full-length Annexin I and ALP (hereinafter, also referred to as an "Annexin I(FL)-ALP conjugate").
Fig. 30 is a graph showing ALP activity of a conjugate of full-length Annexin VI and ALP (hereinafter, also referred to as an "Annexin VI(FL)-ALP conjugate").

### DETAILED DESCRIPTION OF THE PREFERRED EMBODIMENTS

A calibrator generally refers to a reagent containing a standard substance corresponding to a test substance. The concentration of the test substance in the sample is usually unknown, and can be quantitatively measured using a calibrator. The test substance is an extracellular vesicle having a membrane protein and ALP, and ALP activity of the test substance is measured. The standard substance is a substance having essentially the same or similar structure and properties as the test substance. Therefore, the standard substance of the present invention is a conjugate in which a polypeptide having ALP activity and a polypeptide including an amino acid sequence of a membrane protein present on a surface of an extracellular vesicle are covalently bound (hereinafter, also referred to as a "conjugate of the present invention"). The term "polypeptide" refers to a substance in which a plurality of amino acids are bound by peptide bonds, and also includes proteins and fragments thereof.

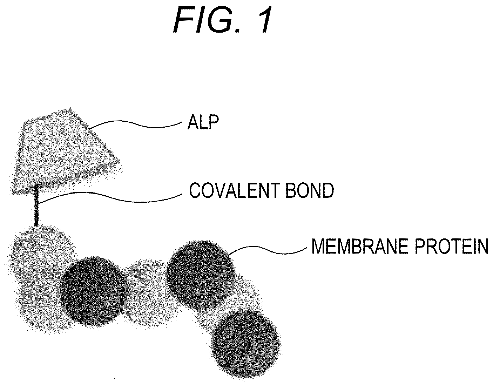

The conjugate of the present invention is composed of a polypeptide having ALP activity and a polypeptide including an amino acid sequence of a membrane protein present on a surface of the extracellular vesicle (hereinafter, also simply referred to as a membrane protein). A schematic diagram of an example of the conjugate is shown in Fig. 1 The polypeptide having ALP activity may be a natural polypeptide or a recombinant polypeptide. The polypeptide including an amino acid sequence of a membrane protein may be a natural polypeptide or a recombinant polypeptide. The amino acid sequence of the polypeptide having ALP activity may be the same amino acid sequence as that of ALP contained in the extracellular vesicle in a sample, or the amino acid sequence may be modified. The amino acid sequence of the polypeptide including an amino acid sequence of a membrane protein may be the same amino acid sequence as that of the membrane protein contained in the extracellular vesicle in a sample, or the amino acid sequence may be modified. The modification of the amino acid sequence of the polypeptide having ALP activity may be deletion, substitution, addition of a part of the amino acid sequence, or a combination thereof, but is a modification in which ALP of the amino acid sequence is not deactivated. The modification of the amino acid sequence of the membrane protein may be deletion, substitution, addition of a part of the amino acid sequence, or a combination thereof, but the amino acid sequence including the modification is preferably an amino acid sequence that can be recognized by the capture antibody described later.

The origin of ALP is not particularly limited, and the ALP may be human-derived ALP or non-human-derived ALP. Examples of the non-human-derived ALP include ALP derived from bovine, mouse, rat, dog, cat, rabbit, and the like, and among them, bovine small intestine-derived ALP (BIAP) is preferable. Examples of the type of ALP include tissue non-specific ALP, small intestine ALP, placenta ALP, and germ cell ALP.

Examples of BIAP include BIAP I, BIAP II, BIAP III, BIAP IV, BIAP V, BIAP VI, BIAP VII, and the like (see Manes T. et al., (1998) J. Biol. Chem., Vol. 273, pp. 23353-23360, U.S. Patent No. 6,406,899, etc.). BIAP may be a commercially available product, and examples thereof include ALP (HighSpecific Activity) manufactured by Oriental Yeast Co., Ltd.

In the conjugate of the present invention, the "membrane protein present on a surface of the extracellular vesicle" may be any membrane protein known to be generally present on the surface of the extracellular vesicle. Examples thereof include CD9, CD63, CD81, CD82, CD53, CD37, Annexin A1 (Annexin I), Annexin A2, Annexin A4, Annexin A5 (Annexin V), Annexin A6 (Annexin VI), Annexin A7, Annexin A11, Pit 1, Pit 2, Sortilin, Rab, Alix, Tsg101, selectin, lactadherin, integrin, flotillin, glycosylphosphatidylinositol, heat-shock protein (HSP-60, HSP-70, HSP-A5, CCT2, HSP90, and the like), matrix metalloproteinases (MMP-2, MMP-3, MMP-9, MMP-13, and the like), S100 protein family (S100-A9 and the like), major histocompatibility complexes (MHC)-I, MHC-II, and the like. The amino acid sequence of a membrane protein present on a surface of the extracellular vesicle may be a partial sequence or a full-length sequence of the membrane protein described above. For example, an amino acid sequence selected from an extracellular domain of CD9, an extracellular domain of CD63, an extracellular domain of CD81, Annexin A1 (Annexin I), Annexin A2, Annexin A4, Annexin A5 (Annexin V), Annexin A6 (Annexin VI), Annexin A7, Annexin A11, Pit 1, Pit 2, sortilin, and S100-A9 is preferable. Amino acid sequences themselves of these membrane proteins are known, and can be acquired from known databases such as database provided by NCBI (National Center for Biotechnology Information). The polypeptide may have a modified amino acid sequence as long as the capture antibody described below is present or can be produced. When the conjugate has a polypeptide including an amino acid sequence of the membrane protein, the conjugate can be captured by a capture antibody capable of binding to the polypeptide. Since this conjugate is designed so as to be able to bind to a capture antibody used for measurement of a membrane protein of the extracellular vesicle, concentration of extracellular vesicles in a sample can be acquired by using a calibrator containing this conjugate.

Specific examples of the conjugate include a conjugate in which an extracellular domain of CD9 and ALP are covalently bound, a conjugate in which an entire region of CD9 and ALP are covalently bound, a conjugate in which an extracellular domain of CD63 and ALP are covalently bound, a conjugate in which an extracellular domain of CD81 and ALP are covalently bound, a conjugate in which an entire region of Annexin I and ALP are covalently bound, a conjugate in which an entire region of Annexin VI and ALP are covalently bound, and the like. The amino acid sequence of the extracellular domain of CD9 is shown in SEQ ID NO: 1. The amino acid sequence of the entire region of CD9 is shown in SEQ ID NO: 2. The amino acid sequence of the extracellular domain of CD63 is shown in SEQ ID NO: 3. The amino acid sequence of the extracellular domain of CD81 is shown in SEQ ID NO: 4. The amino acid sequence of the entire region of Annexin I is shown in SEQ ID NO: 5. The amino acid sequence of Annexin VI/ANXA6 Protein, Human, Recombinant (His Tag), 11161-H08E (Sino Biological), which is a recombinant protein composed of the entire region of Annexin VI (hereinafter, also referred to as Annexin VI(FL)) is shown in SEQ ID NO: 6. SEQ ID NOs: 1 to 6 are shown below.
SEQ ID NO: 1: Amino acid sequence of extracellular domain of CD9 
SHKDEVIKEVQEFYKDTYNKLKTKDEPQRETLKAIHYALNCCGLAGGV
EQFISDICPKKDVLETFTVKSCPDAIKEVFDNKFHI
SEQ ID NO: 2: Amino acid sequence of entire region of CD9 
MPVKGGTKCIKYLLFGFNFIFWLAGIAVLAIGLWLRFDSQTKSIFEQET
NNNNSSFYTGVYILIGAGALMMLVGFLGCCGAVQESQCMLGLFFGFLLVIFAIEI
AAAIWGYSHKDEVIKEVQEFYKDTYNKLKTKDEPQRETLKAIHYALNCCGLAG
GVEQFISDICPKKDVLETFTVKSCPDAIKEVFDNKFHIIGAVGIGIAVVMIFGMIFS
MILCCAIRRNREMV
SEQ ID NO: 3: Amino acid sequence of extracellular domain of CD63 
AGYVFRDKVMSEFNNNFRQQMENYPKNNHTASILDRMQADFKCCGA
ANYTDWEKIPSMSKNRVPDSCCINVTVGCGINFNEKAIHKEGCVEKIGGWLRK
NV
SEQ ID NO: 4: Amino acid sequence of extracellular domain of CD81 
FVNKDQIAKDVKQFYDQALQQAVVDDDANNAKAVVKTFHETLDCCG
SSTLTALTTSVLKNNLCPSGSNIISNLFKEDCHQKIDDLFSGK
SEQ ID NO: 5: Amino acid sequence of Annexin I(FL) 
MAMVSEFLKQAWFIENEEQEYVQTVKSSKGGPGSAVSPYPTFNPSSDV
AALHKAIMVKGVDEATIIDILTKRNNAQRQQIKAAYLQETGKPLDETLKKALTG
HLEEVVLALLKTPAQFDADELRAAMKGLGTDEDTLIEILASRTNKEIRDINRVY
REELKRDLAKDITSDTSGDFRNALLSLAKGDRSEDFGVNEDLADSDARALYEA
GERRKGTDVNVFNTILTTRSYPQLRRVFQKYTKYSKHDMNKVLDLELKGDIEK
CLTAIVKCATSKPAFFAEKLHQAMKGVGTRHKALIRIMVSRSEIDMNDIKAFYQ
KMYGISLCQAILDETKGDYEKILVALCGGN
SEQ ID NO: 6: Amino acid sequence of Annexin VI(FL)

MAKPAQGAKYRGSIHDFPGFDPNQDAEALYTAMKGFGSDKEAILDIIT
SRSNRQRQEVCQSYKSLYGKDLIADLKYELTGKFERLIVGLMRPPAYCDAKEIK
DAISGIGTDEKCLIEILASRTNEQMHQLVAAYKDAYERDLEADIIGDTSGHFQK
MLVVLLQGTREEDDVVSEDLVQQDVQDLYEAGELKWGTDEAQFIYILGNRSK
QHLRLVFDEYLKTTGKPIEASIRGELSGDFEKLMLAVVKCIRSTPEYFAERLFKA
MKGLGTRDNTLIRIMVSRSELDMLDIREIFRTKYEKSLYSMIKNDTSGEYKKTLL
KLSGGDDDAAGQFFPEAAQVAYQMWELSAVARVELKGTVRPANDFNPDADA
KALRKAMKGLGTDEDTIIDIITHRSNVQRQQIRQTFKSHFGRDLMTDLKSEISGD
LARLILGLMMPPAHYDAKQLKKAMEGAGTDEKALIEILATRTNAEIRAINEAYK
EDYHKSLEDALSSDTSGHFRRILISLATGHREEGGENLDQAREDAQVAAEILEIA
DTPSGDKTSLETRFMTILCTRSYPHLRRVFQEFIKMTNYDVEHTIKKEMSGDVR

DAFVAIVQSVKNKPLFFADKLYKSMKGAGTDEKTLTRIMVSRSEIDLLNIRREFI

EKYDKSLHQAIEGDTSGDFLKALLALCGGED

In the conjugate, a polypeptide having ALP activity and a polypeptide including an amino acid sequence of a membrane protein are covalently bound. For example, by mixing and reacting maleimidated ALP and a reduced membrane protein, a covalent bond is formed between a maleimide group of ALP and a thiol group (-SH) of the membrane protein, so that a conjugate can be obtained. Mixing conditions are not particularly limited as long as protein is not denatured. For example, maleimidated ALP and reduced membrane protein may be mixed and stirred or allowed to stand at 4°C to 40°C, preferably 15°C to 37°C.

The covalent bond between the polypeptide having ALP activity and the polypeptide including an amino acid sequence of a membrane protein may be formed by covalently bonding the polypeptide having ALP activity and the polypeptide including an amino acid sequence of a membrane protein using functional groups of each of the polypeptide having ALP activity and the polypeptide including an amino acid sequence of a membrane protein, or may be bonded via a crosslinker. For example, a reaction using a condensing agent or a crosslinker is simple and preferable. Such reactions are known per se. Although the functional group is not particularly limited, a sulfhydryl group, an amino group and a carboxyl group are preferred because commercially available condensing agents and crosslinkers can be used.

The condensing agent is not particularly limited, and when the polypeptide having ALP activity and the polypeptide including an amino acid sequence of a membrane protein are covalently bonded by an amidation reaction, examples of the condensing agent include O-(7-azabenzotriazol-1-yl)-1,1,3,3-tetramethyluronium hexafluorophosphonate, 4-(4,6-dimethoxy-1,3,5-triazin-2-yl)-4-methylmorpholinium chloride, 2-chloro-1,3-dimethylimidazolinium, 1H-benzotriazol-1-yloxytripyrrolidinophosphonium hexafluorophosphate, diphenylphosphoryl azide, chlorotripyrrolidinophosphonium hexafluorophosphate, N,N'-diisopropylcarbodiimide, and the like.

The crosslinker is not particularly limited, and can be appropriately selected according to the functional groups of each of the polypeptide having ALP activity and the polypeptide including an amino acid sequence of a membrane protein.

The compound having a sulfhydryl group as a functional group can be bonded to a compound having a maleimide group or a bromo (or iodo) acetamide group as shown in the figure below. For example, when a polypeptide having ALP activity having a sulfhydryl group and a polypeptide including an amino acid sequence of a membrane protein having a sulfhydryl group are bound to each other, a crosslink reagent having maleimides at both ends may be used as a crosslinker.

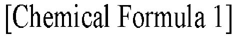

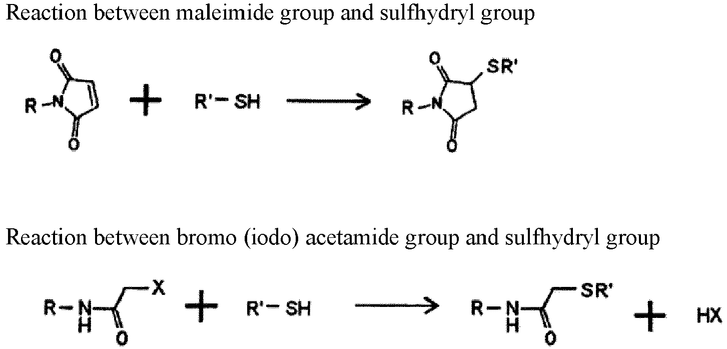

A compound having an amino group as a functional group can be bonded to an N-hydroxysuccinimide (NHS) ester or a compound having an isothiocyano group as shown in the figure below. For example, when a polypeptide having ALP activity having an amino group and a polypeptide including an amino acid sequence of a membrane protein having an amino group are bound to each other, a crosslink reagent having NHS esters at both ends may be used as a crosslinker.

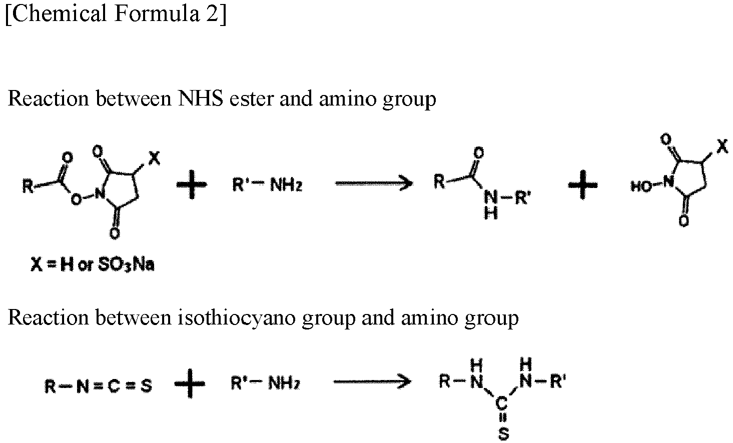

A compound having a carboxy group as a functional group can be bound to a compound having an amino group as a reactive group through three steps shown in the figure below. As shown in the first step in the figure below, a compound having a carboxy group is reacted with a compound having a carbodiimide group (-N=C=N-) (in the figure below, 1-[3-(dimethylamino)propyl]-3-ethylcarbodiimide (WSC)). Next, as shown in the second step in the figure below, the product of the first step is reacted with NHS to form an unstable NHS ester. Then, as shown in the third step in the figure below, the product of the second step and the compound having an amino group can be crosslinked by reacting them. For example, when a polypeptide having ALP activity having a carboxy group and a polypeptide including an amino acid sequence of a membrane protein having an amino group are bound to each other, they can be crosslinked in this manner. When a polypeptide having ALP activity having a carboxy group and a polypeptide including an amino acid sequence of a membrane protein having a carboxy group are bound to each other, a crosslink reagent having amino groups at both ends may be used.

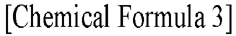

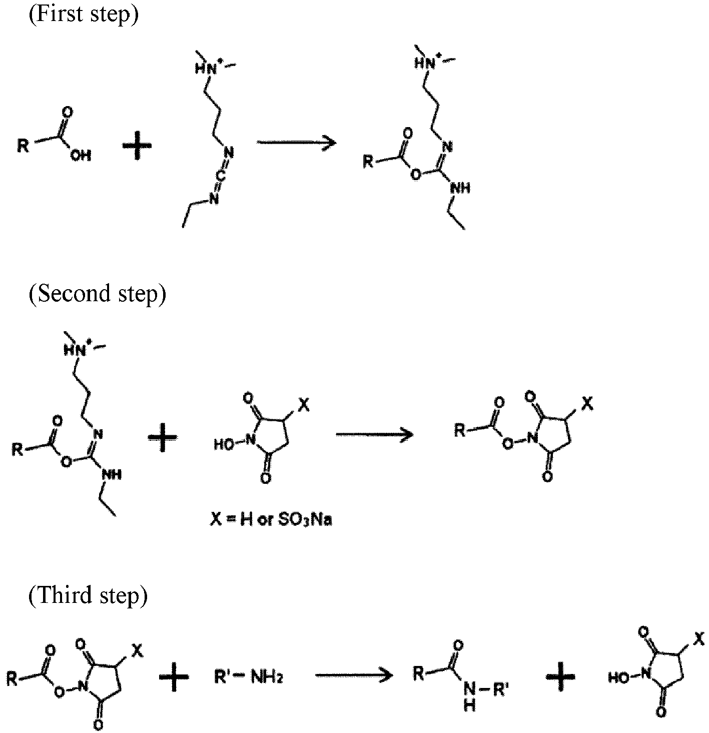

The calibrator may be in a form of solid (for example, powder, crystal, freeze-dried product, or the like) or in a form of liquid (for example, solution, suspension, emulsion, or the like). When the calibrator is a solid, it is usually dissolved in a solvent and used. The solvent is preferably an aqueous solvent, and examples thereof include physiological saline solutions, buffer solutions, and the like. The buffer solution is a buffer solution having a buffering effect at a pH near neutrality (for example, a pH of 6 or more and 8 or less). Examples of the buffer solution include Good buffers such as HEPES, MES, and PIPES, tris buffered saline (TBS), phosphate buffered saline (PBS), and the like.

The calibrator may contain additives. Examples of the additive include protein stabilizers such as bovine serum albumin (BSA), preservatives such as sodium azide, and inorganic salts such as sodium chloride.

The calibrator may be in a form of one reagent or in a form of a reagent set including a plurality of reagents. The calibrator in the form of one reagent includes, for example, one container containing a conjugate of an extracellular domain of CD9 and bovine small intestine-derived ALP.

When the calibrator is a reagent set, the concentrations of the conjugates in a plurality of calibrators are different from each other. The reagent set includes, for example, a plurality of containers containing each of the plurality of calibrators. The plurality of containers contain conjugates at different concentrations.

The number of calibrators included in the reagent set is not particularly limited, and can be selected from, for example, 2, 3, 4, 5, 6 and 7. The concentration of the conjugate in each calibrator is not particularly limited, and it is preferable that a calibration curve described later can be suitably prepared. The conjugate concentration of the calibrator having the lowest conjugate concentration among the plurality of calibrators is preferably 1 µg/mL or more, more preferably 1.25 µg/mL or more, and still more preferably 1.50 µg/mL or more. The conjugate concentration of the calibrator is preferably 10 µg/mL or less, more preferably 5 µg/mL or less, still more preferably 2 µg/mL or less, and most preferably 1.64 µg/mL or less. The calibrator having the highest concentration of the conjugate may contain the conjugate at a concentration equal to or more than 2 times and equal to or less than 1000 times the lowest concentration.

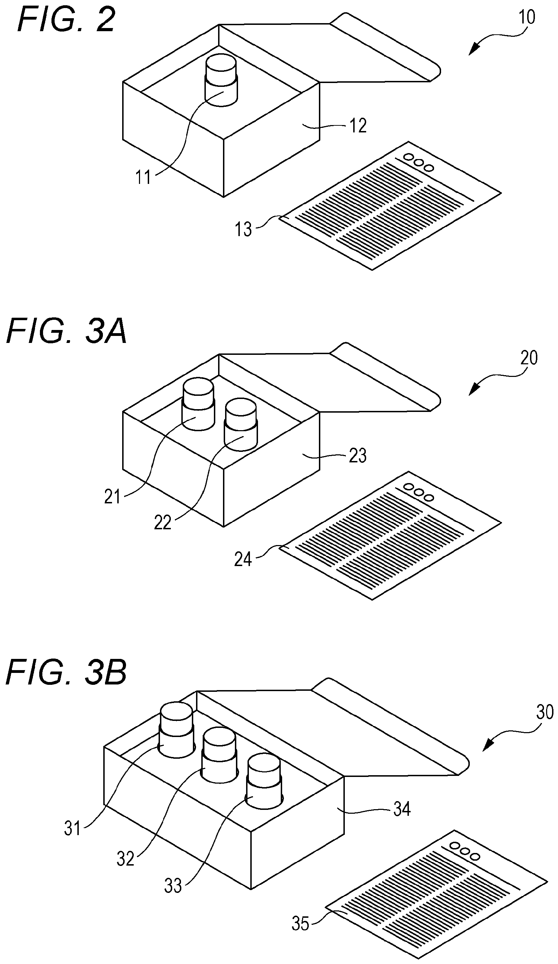

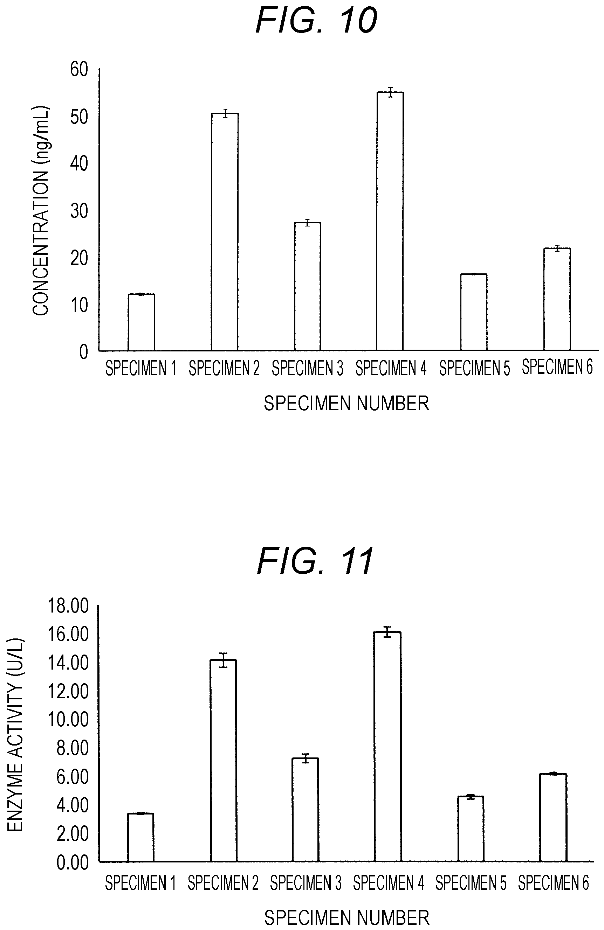

An example of a calibrator in the form of one reagent is shown in Fig. 2. In Fig. 2, 10 denotes a kit including a calibrator, 11 denotes a first container containing the calibrator containing the conjugate of the present embodiment, 12 denotes a packing box, and 13 denotes an attached document.

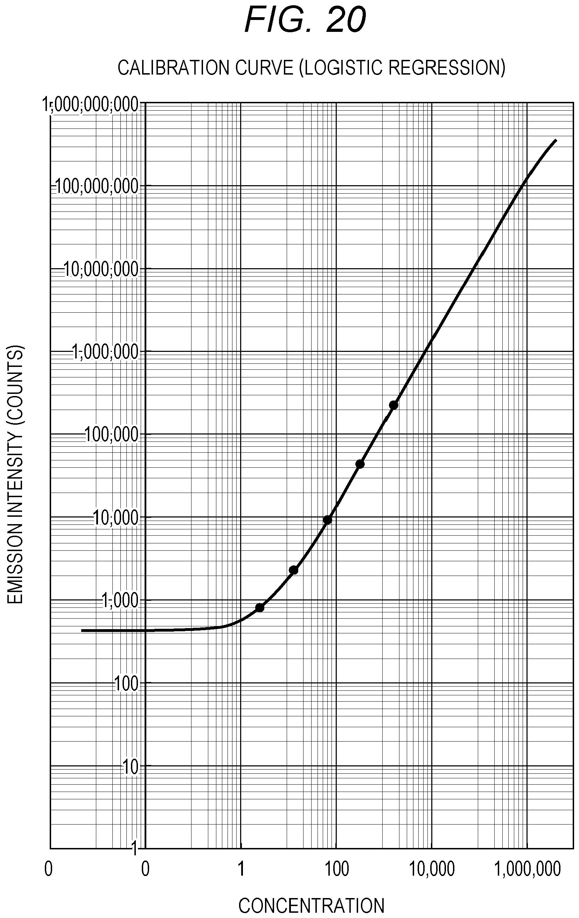

An example of a reagent set including a plurality of calibrators is shown in Fig. 3A. In Fig. 3A, 20 denotes a kit including a calibrator, 21 denotes a first container containing a calibrator containing the conjugate of the present embodiment, 22 denotes a second container containing a calibrator containing a conjugate having a concentration different from that of the calibrator in the first container, 23 denotes a packing box, and 24 denotes an attached document.

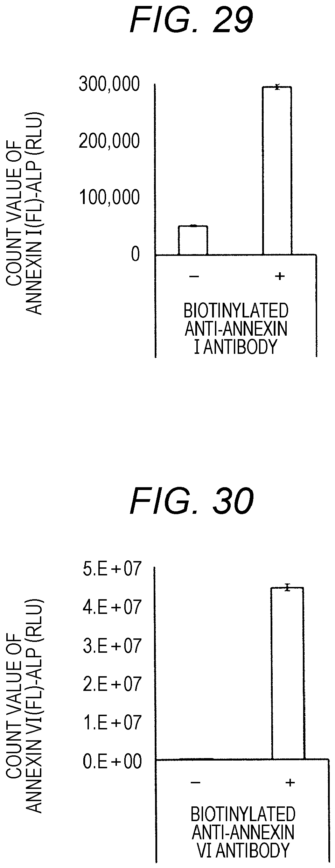

Another example of a reagent set including a plurality of calibrators is shown in Fig. 3B. In Fig. 3B, 30 denotes a kit including a calibrator, 31 denotes a first container containing a calibrator containing the conjugate of the present embodiment, 32 denotes a second container containing a calibrator containing a conjugate having a concentration different from that of the calibrator in the first container, 33 denotes a third container containing a calibrator containing a conjugate having a concentration different from those of the calibrators in the first container and the second container, 34 denotes a packing box, and 35 denotes an attached document.

The reagent set including a plurality of calibrators may separately include a capture antibody for capturing a conjugate in the calibrator or an extracellular vesicle in a sample, a solid phase such as a magnetic particle, a substrate of ALP, and the like in a container. In this case, the reagent set is also a reagent kit for immunoassay. The capture antibody, the solid phase, and the substrate of ALP will be described later. The reagent set may separately include a solution containing no conjugate (negative control) in a container.

In the method of the present invention, an activity value of ALP contained in the extracellular vesicle in a sample is acquired using the calibrator containing the conjugate of the present invention. The activity value of ALP may be a measured value of a signal derived from an enzymatic reaction between the conjugate of the present invention, ALP in the extracellular vesicle in a sample, and a substrate capable of generating a detectable signal, or a value based on the measured value. Examples of the value based on the measured value of the signal include a value obtained by dividing the measured value obtained by optically measuring the signal generated by the enzymatic reaction by a protein amount of (a sample containing) ALP, and the like. Examples of the substrate of ALP include chemiluminescent substrates such as CDP-Star (registered trademark) (disodium 4-chloro-3-(methoxyspiro[1,2-dioxetane-3,2'-(5'-chloro)tricyclo[3.3.1.1^{3,7}]decan]-4-yl)phenyl phosphate) and CSPD (registered trademark) (disodium 3-(4-methoxyspiro[1,2-dioxetane-3,2-(5'-chloro)tricyclo[3.3.1.1^{3,7}]decan]-4-yl)phenyl phosphate), and chromogenic substrates such as 5-bromo-4-chloro-3-indolyl phosphate (BCIP), disodium 5-bromo-6-chloro-indolyl phosphate and p-nitrophenyl phosphate. Among them, CDP-Star is preferably used. Specifically, it can be determined from a measured value obtained by optically measuring p-nitrophenol generated by hydrolyzing p-nitrophenyl phosphate as a substrate with ALP and the protein amount of (a sample containing) ALP.

Examples of the method for measuring enzyme activity of ALP of the conjugate in the calibrator include an immunoassay. Preferably, the immunoassay is an immunoassay using a capture antibody capable of binding to a membrane protein and a substrate of ALP. The type of immunoassay is not particularly limited. For example, an immunoassay from known immunoassay methods such as enzyme-linked immunosorbent assay (ELISA), immunoprecipitation/Western blotting, and immune complex transfer method (see Japanese Laid-Open Patent Publication No. H1-254868) can be used. The immunoassay may be performed by a commercially available immunoassay device such as HISCL (registered trademark) series (Sysmex Corporation).

In the immunoassay, the conjugate in the calibrator is captured by a capture antibody that specifically binds to a membrane protein. The capture antibody that specifically binds to the conjugate refers to an antibody that captures the conjugate on a solid phase by being immobilized on the solid phase. As used herein, the term "antibody" includes full-length antibodies and fragments thereof. Examples of the antibody fragments include Fab, Fab', F(ab')2, Fd, Fd', Fv, light chain, heavy chain variable region (VHH) of heavy chain antibody, reduced IgG (rIgG), one chain antibodies (scFv), and the like.

The capture antibody is selected to specifically bind to the membrane protein in the conjugate. Examples of the capture antibody include an anti-CD9 antibody, an anti-CD63 antibody, an anti-CD81 antibody, an anti-Annexin I antibody, an anti-Annexin VI antibody, an anti-Pit 1 antibody, and the like. Examples of the anti-CD9 antibody include Clone: H19a, 12A1, and the like. Examples of the anti-CD63 antibody include Clone: H5C6 and the like. Examples of the anti-CD81 antibody include Clone: 5A6 and the like. Examples of the anti-Annexin I antibody include Clone: EH17a and the like. Examples of the anti-Annexin VI antibody include Clone: E-5 and the like. Examples of the anti-Pit 1 antibody include Clone: 6A9-F2 and the like. These monoclonal antibodies are commercially available. The capture antibody may be either a monoclonal antibody or a polyclonal antibody, but is preferably a monoclonal antibody.

The conjugate in the calibrator and the capture antibody are bound to form an immune complex. The formation of the immune complex can be performed by mixing the conjugate with the capture antibody. Conditions of temperature and incubation time in the mixing are not particularly limited as long as the conditions are suitable for an antigen-antibody reaction. Such conditions are known per se. For example, the temperature is 4°C or more and 40°C or less, and preferably 20°C or more and 37°C or less. The incubation time can be appropriately determined according to the temperature, and is, for example, 1 minute or more and 24 hours or less. When the contact is performed at a low temperature of 10°C or less, it is preferable to lengthen the time (for example, 1 hour or more).

After forming an immune complex with the conjugate, the capture antibody may be contacted with a solid phase to immobilize the immune complex in the calibrator on the solid phase. Alternatively, a solid phase on which the capture antibody is previously immobilized and the conjugate in the calibrator may be mixed to immobilize the immune complex on the solid phase. The solid phase may be any insoluble carrier capable of immobilizing the capture antibody. The material of the solid phase is not particularly limited. For example, the material can be selected from organic polymer compounds, inorganic compounds, biopolymers, and the like. Examples of the organic polymer compound include latex, polystyrene, polypropylene, and the like. Examples of the inorganic compound include magnetic bodies (iron oxide, chromium oxide, ferrite, and the like), silica, alumina, glass, and the like. Examples of the biopolymer include insoluble agarose, insoluble dextran, gelatin, cellulose, and the like. Two or more of these may be used in combination. The shape of the solid phase is not particularly limited, and examples thereof include microplates, microtubes, test tubes, particles, membranes, and the like. Among them, microplates and particles (particularly magnetic particles) are preferable.

The binding between the solid phase and the capture antibody may be direct or indirect. Examples of the direct binding between the solid phase and the capture antibody include adsorption or covalent binding to the surface of the solid phase by hydrophobic interaction. For example, when the solid phase is an ELISA microplate, the capture antibody can be immobilized in a well of the plate by adsorption. When the solid phase has a functional group on the surface, the capture antibody can be immobilized on the surface of the solid phase by covalent binding using the functional group. For example, when the solid phase is a particle having a carboxy group, a carboxy group on the particle surface is activated with WSC and then reacted with NHS to form an NHS ester. Then, when the particle having the NHS ester is contacted with the capture antibody, the NHS ester reacts with an amino group of the capture antibody, and the capture antibody is covalently immobilized on the particle surface.

Examples of the indirect binding between the solid phase and the capture antibody include binding via a molecule that specifically binds to the capture antibody. By previously immobilizing such a molecule on the surface of the solid phase, the capture antibody can be immobilized on the solid phase. Examples of the molecule that specifically binds to the capture antibody include protein A, protein G, an antibody (a secondary antibody) that specifically recognizes a capture antibody, and the like. A combination of substances interposed between the capture antibody and the solid phase can be used to bind them. Examples of the combination of substances include combinations of any of biotin and its analogs and any of biotin-binding sites, a hapten and an anti-hapten antibody and the like. The biotin and its analogs include biotin and biotin analogs such as desthiobiotin. The biotin-binding sites include avidin and avidin analogs such as streptavidin and tamavidin (registered trademark). For example, when the capture antibody is modified with biotin, the capture antibody can be immobilized on a solid phase on which avidins are immobilized.

The enzyme activity of ALP contained in the immune complex is measured. As described above, the enzyme activity of ALP can be obtained, for example, by optically measuring a signal generated by an enzymatic reaction of ALP. As used herein, the phrase "detecting a signal" includes qualitatively detecting the presence or absence of a signal, quantifying a signal intensity, and semi-quantitatively detecting the signal intensity. The phrase "semi-quantitatively detecting the signal intensity" means to detect the signal intensity in plural stages such as "no signal generated", "weak", "strong", and the like. In the detection of the signal, it is preferable to detect the intensity of the signal quantitatively.

When quantitatively detecting a signal intensity, the signal intensity value itself or a value acquired from the measured value can be used as the activity of ALP. Examples of the value acquired from the measured value of the signal intensity include a value obtained by subtracting the measured value of a negative control sample or the background value from the measured value, and the like. The negative control sample can be appropriately selected, and examples thereof include a sample containing no conjugate, and the like.

B/F (Bound/Free) separation for removing an unreacted free component not forming an immune complex may be performed between formation and detection of the immune complex. The unreacted free component refers to a component not constituting an immune complex. Examples thereof include a capture antibody that does not bind to a conjugate and the like. The means of B/F separation is not particularly limited, and when the solid phase is a particle, B/F separation can be performed by recovering only the solid phase capturing the immune complex by centrifugation. When the solid phase is a container such as a microplate or a microtube, B/F separation can be performed by removing a liquid containing an unreacted free component. When the solid phase is a magnetic particle, B/F separation can be performed by aspirating and removing a liquid containing an unreacted free component by a nozzle while magnetically constraining the magnetic particles with a magnet, which is preferable from the viewpoint of automation. After removing the unreacted free component, the solid phase capturing the immune complex may be washed with a suitable aqueous medium such as PBS.

Since the concentration of the conjugate in the calibrator is known, a calibration curve can be prepared by acquiring the measured values of ALP using a plurality of calibrators having different concentrations and plotting the acquired measured values against the concentration. The calibration curve can be prepared, for example, by plotting the measured values of the conjugates acquired from a plurality of calibrators on an XY plane in which the concentration of the conjugate in the calibrator is taken on an X-axis and the signal values of ALP are taken on a Y-axis to obtain a straight line or a curve by a known method such as a least squares method. By applying the measured value of ALP in the sample to this calibration curve, the concentration of extracellular vesicles containing ALP in the sample can be acquired. Alternatively, the measured values of ALP are acquired using a plurality of calibrators having different concentrations, and an activity value (U/L) of the calibrator is acquired from the acquired measured values. The unit (U) refers to the amount of enzyme that acts on 1 µmol of a substrate per minute. For example, when the U/L of the calibrator is 1 U/L, 1 L of the calibrator contains the amount of enzyme that acts on 1 µmol of a substrate per minute. The calibration curve can also be prepared by plotting the measured values of the conjugates acquired from a plurality of calibrators on an XY plane in which the activity value (U/L) is taken on an X-axis and the signal values of ALP are taken on a Y-axis to obtain a straight line or a curve by a known method such as a least squares method. By applying the measured value of ALP in the sample to this calibration curve, the activity value (U/L) of extracellular vesicles containing ALP in the sample can be acquired.

Conventionally, it has been considered that extracellular vesicle containing hydroxyapatite related to calcification of cells (hereinafter, also referred to as "cEV") does not come out in the blood. The present inventors have found that this cEV is also present in the blood. Then, it has been found that the activity value of ALP present on a surface of the extracellular vesicle in the blood can be used as an index of calcification of a blood vessel. Therefore, it is important to measure the activity value of ALP in the extracellular vesicle in a sample based on a standard substance.

In the method of the present invention, an extracellular vesicle contained in a sample is measured by immobilizing an extracellular vesicle contained in a sample on a solid phase (hereinafter, also referred to as a "first immobilization step"), and measuring a signal generated by contacting the immobilized extracellular vesicle with a substrate of ALP (hereinafter, also referred to as a "first measurement step"), and a conjugate contained in a calibrator is measured by immobilizing a conjugate contained in a calibrator on a solid phase (hereinafter, also referred to as a "second immobilization step"), and measuring a signal generated by contacting the immobilized conjugate with a substrate of ALP (hereinafter, also referred to as a "second measurement step"), thereby acquiring an activity value of ALP in the extracellular vesicle contained in a sample from the measured value obtained in the first measurement step and the measured value obtained in the second measurement step. Each step will be described below.

In the first immobilization step, a sample, a solid phase, and a capture antibody capable of binding to a membrane protein present on a surface of an extracellular vesicle are contacted with each other to form an immune complex of the extracellular vesicle in the sample and the capture antibody on the solid phase. A capture antibody capable of binding to a membrane protein present on a surface of an extracellular vesicle is a capture antibody that specifically binds to the membrane protein which is described above. The solid phase and the capture antibody are as described above. The immune complex may be captured on the solid phase after forming the immune complex, or may be formed by previously binding the capture antibody and the solid phase and then contacting the extracellular vesicle with the capture antibody.

The sample is not particularly limited as long as it contains an extracellular vesicle, but is preferably a biological sample. Examples of the biological sample include blood samples (whole blood, plasma, or serum), urine, lymph, tissue fluid, cerebrospinal fluid, saliva, and the like. Among them, blood samples (whole blood, plasma, or serum) are preferable. When insoluble contaminants such as cells are contained in the sample, impurities may be removed from the sample by a known means such as centrifugal separation and filtration. The sample may be diluted with an appropriate aqueous medium as necessary. The aqueous medium is not particularly limited as long as it does not interfere with the measurement described later. Examples of the aqueous medium include water, physiological saline, a buffer solution, and the like. As the buffer solution, a buffer solution used for the above-described calibrator can be used. When plasma is used as the blood sample, an anticoagulant may be used at the time of blood collection. As the anticoagulant, for example, EDTA potassium salt, EDTA sodium salt, sodium citrate, heparin salt, or the like can be used.

The sample may be pretreated before immobilization with the solid phase. Examples of the pretreatment include removal of impurities by centrifugation or filtration of a sample, immunoprecipitation using an antibody, or the like, addition of an aqueous medium, and the like The aqueous medium is as described above.

In the first measurement step, a signal generated by contacting the extracellular vesicle immobilized in the first immobilization step with a substrate of ALP is measured. The substrate of ALP and the measurement of signal are as described above. The first immobilization step and the first measurement step may be performed for each of a plurality of samples.

In the second immobilization step, a calibrator, a solid phase, and a capture antibody capable of binding to a membrane protein present on a surface of the extracellular vesicle are contacted with each other to form an immune complex of the conjugate in the calibrator and the capture antibody on the solid phase. The calibrator, the conjugate, the solid phase, and the capture antibody are as described above. The immune complex may be captured on the solid phase after forming the immune complex, or may be formed by previously binding the capture antibody and the solid phase and then contacting the extracellular vesicle with the capture antibody. The capture antibodies used in the first immobilization step and the second immobilization step need to be the same.

In the second measurement step, a signal generated by contacting the conjugate immobilized in the second immobilization step with the substrate of ALP is measured. The substrate of ALP and the measurement of signal are as described above.

The calibrators used in the second immobilization step and the second measurement step are preferably a plurality of calibrators having different concentrations. Since the concentration of the conjugate in the calibrator is known, when a plurality of calibrators having different concentrations is used, measured values are each acquired by performing the second immobilization step and the second measurement step on a calibrator having a plurality of concentrations, and a calibration curve can be prepared from the measured values. The concentration of extracellular vesicles in a sample can be calculated by applying the measured value of the signal obtained in the first measurement step to the calibration curve. The activity value of ALP can be acquired from the measured values of protein concentration and enzyme activity.

Whichever of the combination of the first immobilization step and the first measurement step and the combination of the second immobilization step and the second measurement step may be performed first, or they may be performed simultaneously. That is, the second immobilization step and the second measurement step may be performed after the first immobilization step and the first measurement step are performed, or the first immobilization step and the first measurement step may be performed after the second immobilization step and the second measurement step are performed. Alternatively, the first measurement step and the second measurement step may be each performed after the first immobilization step and the second immobilization step are performed.

B/F separation for removing unreacted free components not forming an immune complex may be performed between the first immobilization step and the first measurement step or between the second immobilization step and the second measurement step. The B/F separation is as described above.

A further related aspect of the invention is a conjugate in which a polypeptide having ALP activity and a polypeptide including an amino acid sequence of a membrane protein present on a surface of the extracellular vesicle are covalently bound. The conjugate can be used as a calibrator for measuring the activity of ALP contained in the extracellular vesicle in a sample. The conjugate is as described above.

A further, related aspect of the invention is the use of the conjugate as a calibrator. By using the conjugate as a calibrator, the activity value of ALP contained in the extracellular vesicle in a sample can be obtained. The conjugate and sample are as described above.

A further related aspect of the invention is a method for acquiring information on the concentration of extracellular vesicles in a sample, the method including acquiring a measured value of enzyme activity of ALP contained in an extracellular vesicle in a sample using a calibrator containing a conjugate in which a polypeptide having ALP activity and a polypeptide including an amino acid sequence of a membrane protein present on a surface of the extracellular vesicle are covalently bound. By using a calibrator containing a conjugate in which a polypeptide having ALP activity and a polypeptide including an amino acid sequence of a membrane protein present on a surface of the extracellular vesicle are covalently bound, the protein concentration of the conjugate and the enzyme activity of ALP in the calibrator are measured, and information on the concentration of extracellular vesicles in a sample can be acquired from the measured values of the protein concentration and the enzyme activity. For example, the measured values of the protein concentration and the enzyme activity of ALP are each acquired from a plurality of calibrators, a calibration curve is prepared based on the measured values of the protein concentration and the enzyme activity, and the measured value of the sample is applied thereto, whereby the concentration of extracellular vesicles in a sample can be calculated. In this case, the information on the concentration of extracellular vesicles is the concentration of extracellular vesicles in a sample itself.

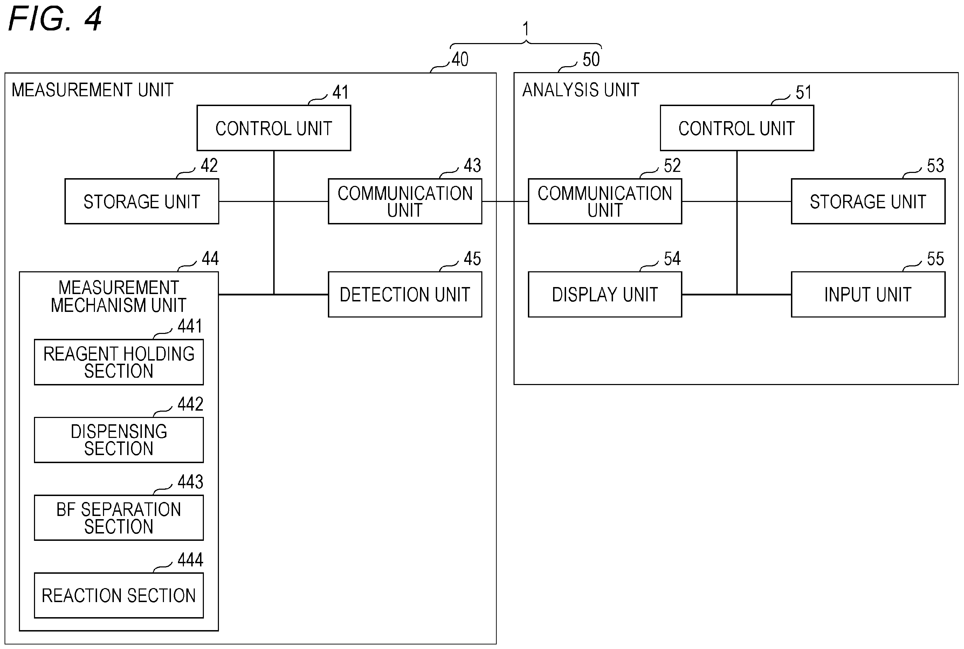

The method of the present invention may be performed by a commercially available immunoassay device. Examples of the immunoassay device include HISCL (registered trademark)-5000, HISCL-2000i, and HI-1000 (all manufactured by Sysmex Corporation), and the like. A block diagram showing a configuration of an exemplary immunoassay device is shown in Fig. 4.

An immunoassay device 1 includes a measurement unit 40 and an analysis unit 50.

The measurement unit 40 includes a control unit 41, a storage unit 42, a communication unit 43, a measurement mechanism unit 44, and a detection unit 45.

The control unit 41 includes, for example, a CPU and its peripheral circuit. The control unit 41 controls an operation of each unit of the measurement unit 40 and the measurement mechanism unit 44 according to each function.

The storage unit 42 includes a hard disk that stores various programs, various data, and the like for the control unit 41 to control each unit of the measurement unit 40.

The communication unit 43 inputs and outputs data to and from an external device under control of the control unit 41. The communication unit 43 includes, for example, an interface circuit using an arbitrary communication standard such as Ethernet (registered trademark) and IEEE1394.

The measurement mechanism unit 44 mainly includes a reagent holding section 441 that holds a reagent, a dispensing section 442 that dispenses the reagent held in the reagent holding section 441 and a measurement target (sample or calibrator) into a reaction container, a BF separation section 443 that executes the BF separation processing described above, and a reaction section 444 that heats the reaction container to promote a reaction between an extracellular vesicle or a conjugate and the reagent.

The detection unit 45 detects a signal generated by the contact of the extracellular vesicle or ALP of the conjugate in the reaction container with the substrate. The detection unit 45 outputs a signal value corresponding to the signal.

The analysis unit 50 mainly includes a control unit 51, a communication unit 52, a storage unit 53, a display unit 54, and an input unit 55.

The control unit 51 controls an operation of each unit of the analysis unit 50 according to each function. The control unit 51 includes, for example, a CPU and its peripheral circuit.

The communication unit 52 is a circuit that inputs and outputs data to and from an external device including the communication unit 43 of the measurement unit 40 under control of the control unit 51. The communication unit 52 includes, for example, an interface circuit using an arbitrary communication standard such as Ethernet (registered trademark) and IEEE 1394, and the like.

The storage unit 53 stores programs executed by the control unit 51, various data, and the like. The storage unit 53 includes a hard disk similarly to the storage unit 42.

The display unit 54 is a touch panel type display including a display device such as a liquid crystal display or an organic EL display.

The input unit 55 is a device that inputs various commands such as a command for instructing preparation of a calibration curve, various data necessary for operating the immunoassay device 1, and the like to the immunoassay device 1. The input unit 55 includes a keyboard, a pointing device such as a mouse or a touch panel, a plurality of input switches to which predetermined functions are assigned, and the like.

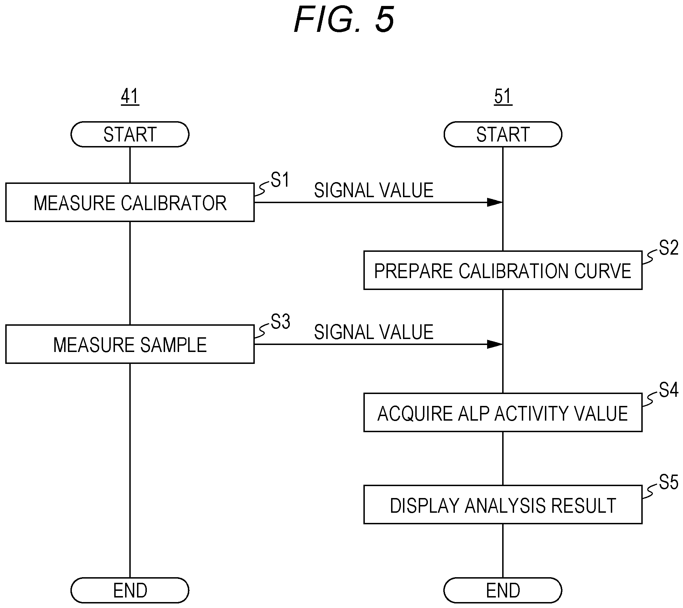

Fig. 5 is a flowchart showing an example of a measurement method using the immunoassay device 1. In step S1, the control unit 41 executes a calibrator measurement step. In the calibrator measurement step, the control unit 41 controls the measurement mechanism unit 44 to immobilize the conjugate contained in the calibrator on the solid phase and contact the conjugate immobilized on the solid phase with the substrate of ALP. In this step, the control unit 41 causes the dispensing section 442 to dispense the reagent and the calibrator held by the reagent holding section 441 into the reaction container. The reagent held by the reagent holding section 441 includes, for example, a capture antibody reagent containing a capture antibody, a solid-phase reagent containing a solid phase composed of a magnetic particle, and a substrate solution containing a chemiluminescent substrate of ALP. First, the control unit 41 causes the dispensing section 442 to dispense the capture antibody reagent, the solid-phase reagent, and the calibrator into the reaction container. As a result, a complex of the conjugate contained in the calibrator and the capture antibody is formed on the solid phase. The control unit 41 causes the BF separation section 443 to remove unreacted components in the reaction container. Next, the control unit 41 causes the dispensing section 442 to dispense the substrate solution into the reaction container. This contacts the conjugate on the solid phase with the substrate of ALP. The reaction container is heated by the reaction section 444 for a predetermined time. Next, the control unit 41 causes the detection unit 45 to detect a signal generated by contact between the conjugate and ALP. The detection unit 45 outputs a signal value corresponding to the detected signal, and the control unit 41 stores the output signal value in the storage unit 42. The control unit 41 transmits the signal value stored in the storage unit 42 to the analysis unit 50 via the communication unit 43. The control unit 51 stores the signal value received via the communication unit 52 in the storage unit 53. The processing in step S1 is repeated by the number of calibrators supplied to the measurement unit 40.

In step S2, the control unit 51 executes a calibration curve preparation step. In the calibration curve preparation step, the control unit 51 prepares a calibration curve based on the signal values received from the measurement unit 40 and stored in the storage unit 53. The method for preparing a calibration curve is as described above. The control unit 51 stores the prepared calibration curve in the storage unit 53.

In step S3, the control unit 41 executes a sample measurement step. In the sample measurement step, the control unit 41 controls the measurement mechanism unit 44 in the same manner as the processing described in step S1 to immobilize the extracellular vesicle contained in a sample on a solid phase and contact the extracellular vesicle immobilized on the solid phase with the substrate of ALP. The control unit 41 causes the detection unit 45 to detect a signal generated by contact between the extracellular vesicle and ALP. The detection unit 45 outputs a signal value corresponding to the detected signal, and the control unit 41 stores the output signal value in the storage unit 42. The control unit 41 transmits the signal value stored in the storage unit 42 to the analysis unit 50 via the communication unit 43. The control unit 51 stores the signal value received via the communication unit 52 in the storage unit 53.

In step S4, the control unit 51 executes an ALP activity value acquisition step. In this step, the control unit 51 acquires an activity value of ALP in the extracellular vesicle in a sample by applying the signal value obtained from the sample to the calibration curve stored in the storage unit 53. The control unit 51 stores the obtained ALP activity value in the storage unit 53.

In step S5, the control unit 51 executes an analysis result display step. The control unit 51 displays the ALP activity value stored in the storage unit 53 on the display unit 54 as a result of the sample measurement.

Hereinbelow, the present invention will be described in detail by examples, but the present invention is not limited to these examples.

### EXAMPLES

### Example 1

A conjugate in which a membrane protein derived from an extracellular vesicle and ALP were bound was prepared as follows, and properties thereof were evaluated.

### [Preparation of calibrator containing CD9(ECD)-ALP]

CD9 Protein, Human, Recombinant (His Tag), 11029-H08H, Sino Biological was used as a polypeptide including an amino acid sequence of a membrane protein. Hereinafter, this polypeptide is also referred to as "CD9(ECD)". CD9(ECD) was a recombinant protein composed of an extracellular domain of CD9. The amino acid sequence of CD9(ECD) was shown in SEQ ID NO: 1.

As a polypeptide having ALP activity, bovine small intestine-derived ALP (HighSpecific Activity) (Oriental Yeast Co., Ltd.) was used. Hereinafter, this polypeptide is also referred to as "BIAP". CD9(ECD) and BIAP were covalently bound via a linker to obtain a calibrator containing a conjugate (CD9(ECD)-ALP). Specifically, the calibrator was obtained as follows.

### (i) Reduction of CD9(ECD)

Using NAP5, 200 µL of CD9(ECD) having a concentration of 0.25 mg/mL was replaced with a reduction treatment buffer solution (pH 6.0, 100 mM sodium phosphate, 150 mM NaCl and 1 mM EDTA) to prepare a CD9(ECD) solution. 2-Mercaptoethylamine (MEA) was added to the reduction treatment buffer solution so as to have a concentration of 350 mM to prepare a MEA solution. The MEA solution was added to 200 µL of the CD9(ECD) solution so as to have a final concentration of MEA of 35 mM, and the mixture was reacted at 37°C for 60 minutes to reduce CD9(ECD). The mixed solution after the reaction was subjected to a desalting column, and CD9(ECD) was recovered.

### (ii) Maleimidation of ALP

100 µL of ALP having a concentration of 10 mg/mL was replaced with a maleimidation buffer solution (pH 7.0, 100 mM TEA, 150 mM NaCl and 1 mM MgClz, 0.1 mM ZnCh) using NAP5 to prepare an ALP solution. N-(6-Maleimidocaproyloxy)succinimide (EMCS) was added to N,N-dimethylformamide (DMF) at 5 mg/mL to prepare an EMCS solution. The EMCS solution was mixed with the ALP solution so that the molar ratio of ALP and EMCS was ALP: EMCS = 1: 10, and reacted at 37°C for 60 minutes to maleimidize ALP. The mixed solution after the reaction was subjected to a desalting column, and ALP was recovered.

### (iii) Binding of CD9(ECD) and ALP

70 µl of CD9(ECD) (0.24 mg/mL of CD9(ECD) concentration) after the reduction treatment of the above (i) and 70 µL of maleimidated ALP (3.24 mg/mL of ALP concentration) of (ii) were mixed and left at 4°C overnight to combine CD9(ECD) and ALP, thereby preparing CD9(ECD)-ALP. This CD9(ECD)-ALP was fractionated by gel filtration chromatography using ACTA (registered trademark) FPLC (Cytiva) using Superdex 200 (Cytiva) as a column.

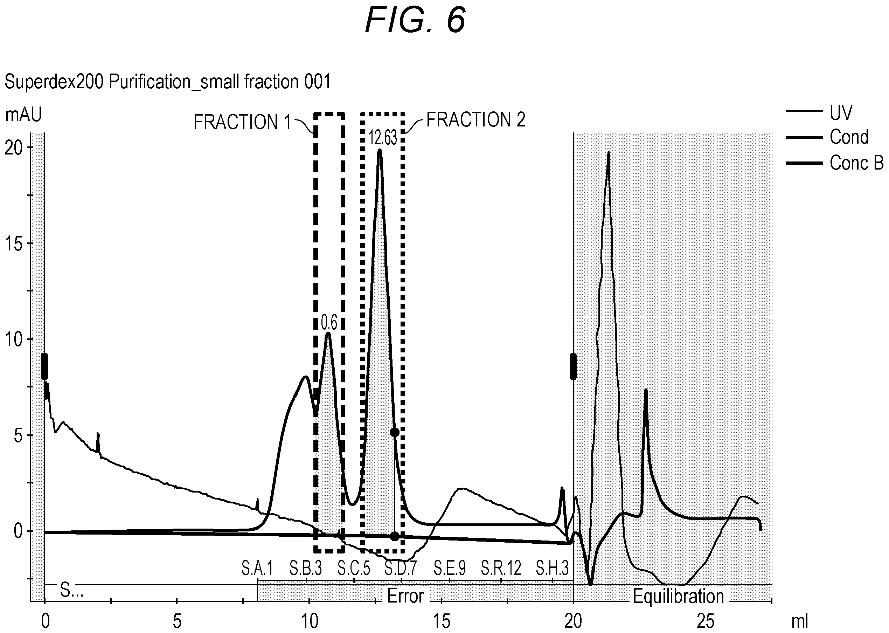

The fractionation results are shown in Fig. 6. Among the recovered fractions, the ALP activity described later was obtained from Fraction 1 of Fig. 6, and thus this fraction was used in the subsequent experiment. In this way, a calibrator containing CD9(ECD)-ALP was obtained. Since the ALP activity described later was also obtained from a fraction of Fraction 2 in Fig. 6, it was considered that Fraction 2 could also be used as a calibrator containing CD9(ECD)-ALP.

### (Measurement of ALP activity)

For the conjugate of Example 1, ALP activity was measured using HI-1000. R1 reagent (capture antibody reagent) for measuring ALP activity was prepared as follows.

As the capture antibody, a commercially available anti-CD9 antibody (Clone: H19a) was used. A 0.1 M EDTA solution (pH 6.0) was added to the capture antibody so as to have an EDTA concentration of 1 mM to prepare a capture antibody solution. A 0.3 M 2-mercaptoethylamine (MEA) solution was added to the capture antibody solution, the mixture was allowed to stand at 37°C for 60 minutes to reduce a disulfide bond of the antibody. The resulting solution was replaced with a gel filtration buffer solution using AmiconUltra 30K. Biotin-PEACs-Maleimide was added to DMSO so as to be 585.16 g/mol, and further diluted 5-fold with a gel filtration buffer solution. A biotin solution was mixed with the reduced antibody solution so that the molar ratio of antibody and biotin was antibody: biotin = 1: 10, and the mixed solution was reacted overnight at 4°C. The next day, the resulting solution was replaced with PBS using AmiconUltra 30K, and a biotin antibody preservation buffer solution was added to prepare a biotin-labeled antibody solution. This biotin-labeled capture antibody solution was added to an R1 reagent buffer solution having the composition shown in Table 1 below so that the antibody concentration was 1 µg/mL to prepare an R1 reagent. As a control, an R1 solution containing no capture antibody was also prepared.

**[Table 1]**

| R1 Reagent buffer solution | |
|---|---|
| Composition | |
| Tris-HCl pH7.5 | 0.1 M |
| NaCl | 150 mM |
| MgCl₂·6H₂O | 10 mM |
| ZnCl₂ | 0.1 mM |
| BSA(Fatty acid free) | 1% |
| Casein sodium | 0.5% |

As an R2 reagent (solid phase), a HISCL R2 reagent (Sysmex Corporation) containing streptavidin-coupled magnetic particles was used. As an R4 reagent (measurement buffer solution), one obtained by removing levamisole from a HISCL R4 reagent (Sysmex Corporation) was used. As an R5 reagent (substrate solution), a HISCL R5 reagent (Sysmex Corporation) containing CDP-star (trademark) (Applied Biosystems) as a chemiluminescent substrate of ALP was used.

Measurement was performed by HI-1000 using the above reagent. This measurement is based on sandwich ELISA on magnetic particles. Specific operations are as follows. Each conjugate (20 µL) was added to the R1 reagent (110 µL) and mixed, and then the R2 reagent (30 µL) was added and mixed. The magnetic particle in the obtained mixed solution was magnetically collected to remove the supernatant, and a HISCL washing solution (300 µL) was added to wash the magnetic particle. The supernatant was removed, and the R4 reagent (50 µL) and the R5 reagent (100 µL) were added to the magnetic particles and thoroughly mixed, and chemiluminescence intensity was measured. The measurement was performed three times for each conjugate, and the average thereof was taken. As a control, the R1 solution containing no capture antibody was also treated and measured in the same manner.

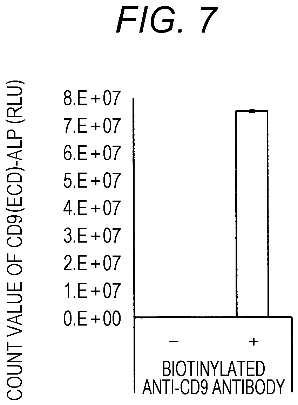

The measurement results are shown in Fig. 7. As shown in Fig. 7, it was found that an antibody-dependent signal can be acquired from the CD9(ECD)-ALP conjugate. Therefore, it was shown that CD9(ECD)-ALP can be used as a standard substance.

### (Evaluation as calibrator)

Properties of the prepared CD9(ECD)-ALP conjugate were evaluated. The calibrator was valued by ALP of cEV derived from a calcified cell. Preparation of calcified extracellular vesicle from the calcified cell was carried out by the following process.

### (Preparation of calcification-induced cell)

Calcification of human osteosarcoma derived cell line Saos-2 cell (hereinafter referred to as Saos-2 cell) was induced to prepare a calcified cell. It is known that the Saos-2 cell can undergo osteogenic differentiation into an osteoblast-like cell under specific culture conditions, and its extracellular vesicle contains ALP. The Saos-2 cell was purchased from RIKEN BioResource Research Center (RIKEN BRC). The cell was cultured according to the RIKEN BRC culture method, with use of McCoy's 5A medium (Sigma-Aldrich) containing 15% FBS, in 5% CO₂ at 37°C.

The Saos-2 cell was transferred to αMEM medium (GIBCO) containing 10% FBS and cultured until confluent. Then, the medium was changed to a calcification induction medium (αMEM medium with 10% FBS, 10 mM HEPES (Nacalai Tesque), 50 µg/mL ascorbate 2-phosphate (Sigma-Aldrich) and 1.8 mM potassium dihydrogen phosphate (FUJIFILM Wako Pure Chemical Corporation)), and the cells were cultured for 14 days to induce transformation into osteoblast-like cells. The medium was changed once every two or three days until Day 10 after the change to the calcification induction medium, and the cells were cultured in the same medium from Day 10 to Day 14. After the culture, the Saos-2 cells and the culture supernatant were recovered. The recovered Saos-2 cells were immobilized with 4% paraformaldehyde (PFA) at 4°C for 10 minutes. The immobilized Saos-2 cells were washed three times with PBS, and stained with Alizarin Red using 50 mM Alizarin Red (pH 4.2) at room temperature for 10 minutes to confirm transformation into osteoblast-like cells.

### (Preparation of cEV)

The culture supernatant of Saos-2 cells was subjected to centrifugation at 300 g for 10 minutes and further centrifuged at 2,000 g for 10 minutes to recover the supernatant. The supernatant was transferred to an ultracentrifuge tube and centrifuged at 100,000 g for 20 minutes. After centrifugation, a precipitate in the ultracentrifuge tube was collected and suspended in a phosphate buffer solution containing 25 mM trehalose to prepare a sample containing cEV (hereinafter, also referred to as a "cEV sample"). The cEV sample was stored at -80°C.

### (Valuing of protein concentration)

The protein concentration of the CD9(ECD)-ALP conjugate was valued using the cEV sample. First, the absorbance (A280) of the cEV sample described above was measured, and the protein concentration (ng/mL) was quantified. Then, the cEV sample was measured by HI-1000 using the above-described R1, R2, R4, and R5 reagents, and count values were acquired.

The CD9(ECD)-ALP conjugate and the cEV sample were suspended in a diluent for calibrator having the composition shown in Table 2 below. Hereinafter, the solution suspended in a diluent for calibrator is referred to as a stock solution of each solution. Some of them were diluted with a diluent for calibrator to prepare various diluted solutions. For each prepared diluted solution, ALP activity was measured in the same manner as described above using HI-1000, and count values were acquired.

**[Table 2]**

| Diluent for calibrator | |
|---|---|
| Composition | |
| Tris-HCl pH7.5 | 0.1 M |
| NaCl | 150 mM |
| MgCl₂·6H₂O | 10 mM |
| BSA(Fatty acid free) | 1% |

From a correspondence relationship between the count values of cEV samples and the protein concentrations obtained, the protein concentration of the CD9(ECD)-ALP conjugate was calculated assuming that the CD9(ECD)-ALP conjugate has the same protein concentration when the count value is the same as that of the cEV sample.

A part of the CD9(ECD)-ALP conjugate stock solution was fractionated and diluted based on a weight method using a diluent for calibrator to prepare nine types of primary calibrators (C0, C0.5, C1, C1.5, C2, C3, C4, C5, C6. C0 is a diluent for calibrator only). In the primary calibrators, the protein concentrations were each calculated from the protein concentration of the stock solution according to the dilution rate. The results are shown in Table 3. Referring to the concentration range of the protein concentration of these primary calibrators, a part of the CD9(ECD)-ALP conjugate stock solution was diluted again using a diluent for calibrator to prepare six types of secondary calibrators (C0, C1, C2, C3, C4, and C5. C0 is a diluent for calibrator only). The protein concentrations (ng/mL) of six types of secondary calibrators are shown in Table 4 below. As shown in Table 4, the protein concentrations of the secondary calibrators varied stepwise from C0 to C5.

**[Table 3]**

| Protein concentration of primary calibrator | | | | | | |
|---|---|---|---|---|---|---|
| | C0 | C1 | C2 | C3 | C4 | C5 |
| CD9(ECD)-ALP conjugate | 0 | 1.52 | 4.89 | 15.65 | 50.05 | 159.87 |

| | | | | | | |
|---|---|---|---|---|---|---|
| Each numerical value represents ng/mL. | | | | | | |

**[Table 4]**

| Protein concentration of secondary calibrator | | | | | | |
|---|---|---|---|---|---|---|
| | C0 | C1 | C2 | C3 | C4 | C5 |
| CD9(ECD)-ALP conjugate | 0 | 1.52 | 6.05 | 23.89 | 207.23 | 645.1 |

| | | | | | | |
|---|---|---|---|---|---|---|
| Each numerical value represents ng/mL. | | | | | | |

### (Valuing of ALP activity value)

Biochemical ALP measurement was performed on secondary calibrators C0 to C5 of a CD9(ECD)-ALP conjugate. Hitachi automatic analyzer 7180 (Hitachi High-Technologies Corporation) was used as a measuring device, Iatro (registered trademark) ALP-IF R1/R2 (LSI Medience Corporation) was used as a measurement reagent, and enzyme calibrator plus (Sysmex Corporation) was used as a calibrator. The measurement results are shown in Table 5 below. As shown in Table 5, the ALP activity values of the secondary calibrators varied stepwise from C0 to C5.

**[Table 5]**

| | C1 | C2 | C3 | C4 | C5 |
|---|---|---|---|---|---|
| CD9(ECD)-ALP conjugate | 1.02 | 3.84 | 14.32 | 129.62 | 387.01 |

| | | | | | |
|---|---|---|---|---|---|
| Each numerical value represents U/L. | | | | | |

### (Measurement of specimen sample using calibrator)

Measurement of a specimen sample was performed using the CD9(ECD)-ALP conjugate.

### (i) Preparation of calibration curve of protein concentration

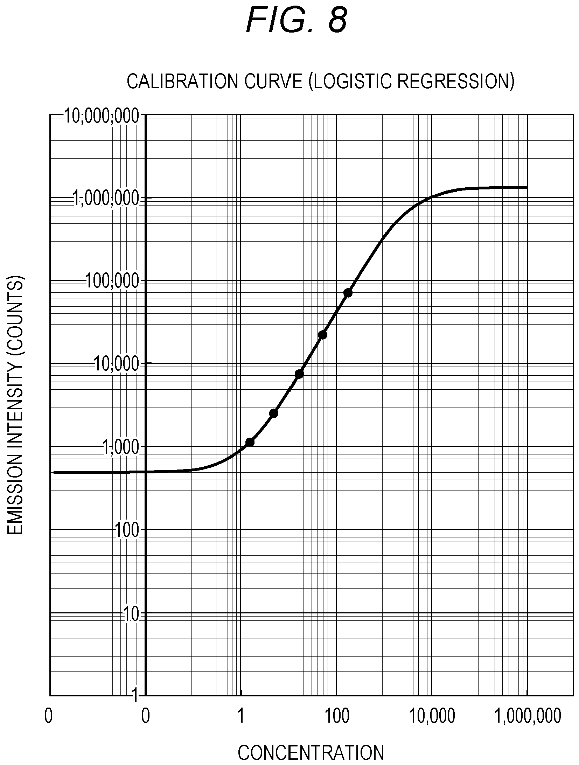

The primary calibrators C0 to C5 of a CD9(ECD)-ALP conjugate in which the protein concentration was valued were measured using the R1, R2, R4 and R5 reagents including the R1 reagent prepared using an anti-CD9 antibody, and HI-1000, and count values were acquired for each calibrator. The measurement was performed three times for each sample. Based on the obtained count values, a calibration curve (calibration curve 1) was prepared based on logistic regression. The measurement results are shown in Table 6 and Fig. 8. In the table, av represents an average value, sd represents a standard deviation, and cv represents a coefficient of variation. From Table 6 and Fig. 8, it was shown that a calibration curve can be prepared using the primary calibrators C0 to C5 of a CD9(ECD)-ALP conjugate in which the protein concentration was valued.

**[Table 6]**

| | C0 | C1 | C2 | C3 | C4 | C5 |
|---|---|---|---|---|---|---|
| Protein concentration (ng/mL) | 0 | 1.52 | 4.89 | 15.65 | 50.05 | 159.87 |
| Count value | 476 | 1075 | 2614 | 7340 | 22367 | 70381 |
| | 492 | 1104 | 2523 | 7234 | 21756 | 67557 |
| | 492 | 1124 | 2542 | 6983 | 22622 | 71563 |
| av | 487 | 1101 | 2560 | 7186 | 22248 | 69834 |
| sd | 8 | 20 | 39 | 150 | 363 | 1681 |
| cv | 2% | 2% | 2% | 2% | 2% | 2% |

The protein concentrations obtained by applying the count values obtained in the above measurement to the calibration curve 1 are shown in Table 7 below. From Table 7, it is found that the values of the protein concentrations acquired by applying the count values to the calibration curve 1 are almost the same as the values of the protein concentrations shown in Table 6. From these, it was shown that the calibration curve can be prepared using the CD9(ECD)-ALP conjugate, and this conjugate can be used as a calibrator.

**[Table 7]**

| | C0 | C1 | C2 | C3 | C4 | C5 |
|---|---|---|---|---|---|---|
| Protein concentration (ng/mL) | 0 | 1.45 | 5.09 | 15.97 | 50.11 | 161.45 |
| | 0.01 | 1.52 | 4.87 | 15.73 | 48.72 | 154.74 |
| | 0.01 | 1.57 | 4.92 | 15.16 | 50.69 | 164.27 |
| av | 0.01 | 1.51 | 4.96 | 15.62 | 49.84 | 160.16 |
| sd | 0 | 0 | 0 | 0 | 1 | 4 |
| cv | 71% | 3% | 2% | 2% | 2% | 2% |
| Regression rate | - | 99% | 101% | 100% | 100% | 100% |

### (ii) Preparation of calibration curve of ALP activity value

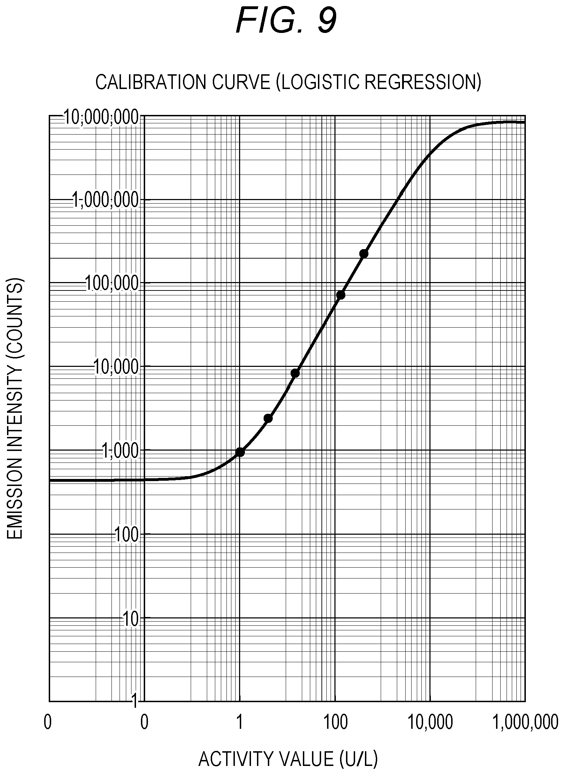

The secondary calibrators C0 to C5 of a CD9(ECD)-ALP conjugate in which the ALP activity value was valued were measured using the R1, R2, R4 and R5 reagents including the R1 reagent prepared using an anti-CD9 antibody, and HI-1000, and count values were acquired for each calibrator. The measurement was performed three times for each sample. Based on the obtained count values, a calibration curve (calibration curve 2) was prepared based on logistic regression. The measurement results are shown in Table 8 and Fig. 9. From Table 8 and Fig. 9, it was shown that a calibration curve can be prepared using the secondary calibrators C0 to C5 of a CD9(ECD)-ALP conjugate in which the ALP activity value was valued.

**[Table 8]**

| | C0 | C1 | C2 | C3 | C4 | C5 |
|---|---|---|---|---|---|---|
| Activity value (U/L) | 0 | 1.02 | 3.84 | 14.32 | 129.62 | 387.01 |
| Count value | 426 | 975 | 2421 | 7977 | 71000 | 219720 |
| | 456 | 937 | 2299 | 8244 | 69423 | 218065 |
| | 461 | 912 | 2454 | 8061 | 69491 | 208881 |
| av | 448 | 941 | 2391 | 8094 | 69971 | 215555 |
| sd | 15 | 26 | 67 | 111 | 728 | 4768 |
| cv | 3% | 3% | 3% | 1% | 1% | 2% |

The ALP activity values obtained by applying the count values obtained in the above measurement to the calibration curve 2 is shown in Table 9 below. The cEVs in Table 9 refer to results similarly performed on a sample in which the protein concentration of the cEV sample was adjusted to 500 ng/ml (based on absorbance A280). From Table 8, it is found that the ALP activity values acquired by applying the count values to the calibration curve 2 are almost the same as the ALP activity values shown in Table 8. From these, it was shown that the calibration curve can be prepared using the CD9(ECD)-ALP conjugate, and this conjugate can be used as a calibrator.

**[Table 9]**

| | C0 | C1 | C2 | C3 | C4 | C5 | cEV |
|---|---|---|---|---|---|---|---|
| Activity value (LT/L) | 0 | 1.04 | 4.17 | 15.13 | 125.9 | 403.11 | 88.32 |
| | 0.02 | 0.97 | 3.84 | 14.37 | 126.24 | 391.97 | 87.17 |
| | 0.06 | 0.96 | 3.81 | 16.64 | 123.89 | 377.68 | 89.22 |
| av | 0.03 | 0.99 | 3.94 | 14.71 | 125.34 | 390.92 | 88.24 |
| sd | 0.03 | 0.04 | 0.16 | 0.31 | 1.04 | 10.41 | 0.84 |
| cv | 89 | 4% | 4% | 2% | 1% | 3% | 1% |
| Regression rate | - | 97% | 103% | 103% | 97% | 101% | |

### (iii) Quantification of protein concentration using specimen sample

The protein concentrations of extracellular vesicles containing ALP were measured using actual specimen samples. As the specimen samples, plasma specimen samples (6 specimens) of patients with history of cardiovascular disease were used. An experiment was performed on a sample obtained by thawing each frozen specimen sample and then diluting the sample 4-fold with a diluent for calibrator. Each prepared specimen sample was measured by HI-1000 using the above R1, R2, R4 and R5 reagents, and count values were acquired. The measurement was performed three times for each specimen sample. The protein concentrations of extracellular vesicles containing ALP contained in the specimen samples were measured by applying the obtained measured values to the calibration curve 1. The measurement results are shown in Fig. 10. From Fig. 10, it can be seen that the protein concentrations of extracellular vesicles containing ALP in the plasma specimen samples can be measured, and the obtained count values can be applied to the calibration curve. From this, it was shown that the protein concentrations of the specimen samples can be quantified using the calibration curve prepared using the CD9(ECD)-ALP conjugate.

### (iv) Quantification of ALP activity value using specimen sample

The count values obtained in the above (iii) were applied to the calibration curve 2 to calculate ALP activity values of specimen samples. The results are shown in Fig. 11. From Fig. 11, it can be seen that the ALP activity values of extracellular vesicles containing ALP in the plasma specimens can be measured, and the obtained count values can be applied to the calibration curve. From this, it was shown that the ALP activity values of specimen samples can be quantified using the calibration curve prepared using the CD9(ECD)-ALP conjugate.

### (v) Comparison with plasma specimen of healthy person

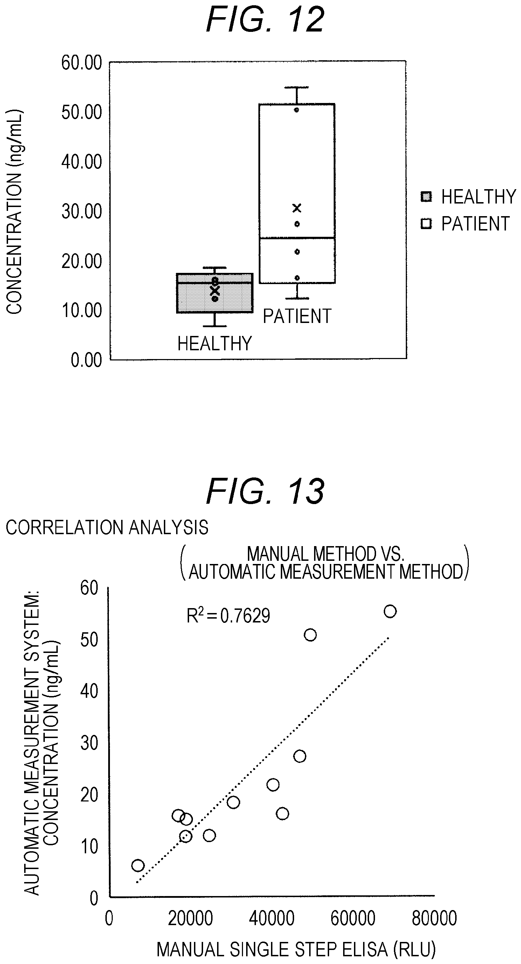

The protein concentrations of extracellular vesicles containing ALP contained in plasma specimen samples (5 specimens) of healthy persons were also determined by the same method as in the above (iii). The results are shown in Fig. 12. Fig. 12 also describes the protein concentrations of extracellular vesicles containing ALP of patients with history of cardiovascular disease described above. From Fig. 12, it can be seen that the protein concentrations of extracellular vesicles containing ALP are increased in the patients with history of cardiovascular disease as compared with those in the healthy persons. As described above, it was shown that the protein concentrations of extracellular vesicles containing ALP can be compared between specimens using the conjugate of the present invention as a calibrator.

### (Comparison between automatic measurement method and measurement by manual method)

For the plasma specimen samples of the patients with history of cardiovascular disease and the plasma specimen samples of the healthy persons described above, the protein concentrations of extracellular vesicles containing ALP were measured by a manual method using ELISA method instead of HI-1000. The ELISA method was performed as follows.

A 96-well plate (Nunc, high binding ELISA plate, #436110) was washed twice with 50 mM Tris-HCl (pH 7.5). The anti-CD9 antibody was adjusted to a concentration of 5 µg/mL with 50 mM Tris-HCl (pH 7.5). This antibody solution was added to the 96-well plate at 50 µL/well and allowed to stand overnight at 4°C to immobilize the antibody in the wells. After removing the antibody solution from the plate, the plate was washed three times with 5 mM phosphate buffer solution. After washing, 200 µL/well of a blocking buffer solution having the composition shown in Table 10 below was each added, and the plate was incubated at room temperature for 2 hours and 30 minutes with stirring on a shaker at 600 rpm to perform blocking. After blocking, the solution was removed, and each plasma sample diluted 2-fold with a diluent for calibrator was added in an amount of 100 µL/well, and the plate was incubated at room temperature for 2 hours at 600 rpm to perform an antigen-antibody reaction. After discarding the solution in the wells after the reaction, each well was washed 6 times using 300 µL/well of HISCL washing solution. After washing, 100 µL/well of R5 reagent was added, and the plate was incubated at room temperature for 20 minutes, then chemiluminescence was detected on a plate reader (TECAN, Infinite Pro200), and ALP activity was measured.

**[Table 10]**

| Composition | |
|---|---|
| TEA buffer pH7.4 | 0.1 M |
| NaCl | 150 mM |
| Sodium caseinate | 0.5% |
| BSA(ChonAnalog) | 1% |

Fig. 13 shows a plot obtained by plotting the protein concentrations obtained by applying the measurement results with HI-1000 for the plasma specimen samples of the patients with history of cardiovascular disease and the plasma specimen samples of the healthy persons to the calibration curve 1 on the Y-axis and the measurement results by ELISA on the X-axis. From Fig. 13, it was found that there is a high correlation between the measurement by the automatic measurement method and the measurement by manual method, and it was shown that the calibrator of the present invention can be used for both the automatic measurement method and the manual method.

### [Stability test of CD9(ECD)-ALP conjugate]

Nine types of primary calibrators (C0, C0.5, C1, C1.5, C2, C3, C4, C5, C6) of a CD9(ECD)-ALP conjugate were dispensed, each stored at -80°C, and thawed after 1 day, 5 days, 7 days, and 26 days, respectively, and each calibrator was measured by HI-1000 using the above R1, R2, R4, and R5 reagents including the R1 reagent, and count values were acquired. The measurement was performed three times for each calibrator. The measurement results are shown in Table 11. In Table 11, the measurement result on Day 1 is 100%. As a comparison, samples obtained by diluting the cEV sample with a diluent for calibrator was similarly performed. The results are shown in Table 12. In Table 12, the measurement result on Day 0 is 100%. Tables 11 and 12 show that the primary calibrators have no large variation in the count values due to storage, whereas the samples obtained by diluting the cEV sample with a diluent for calibrator have a large variation in the count values due to storage. From this, it can be seen that the CD9(ECD)-ALP conjugate has high stability, and is excellent as a calibrator.

**[Table 11]**

| | C0 | C0.5 | C1 | C1.5 | C2 | C3 | C4 | C5 | C6 |
|---|---|---|---|---|---|---|---|---|---|
| Day 1 | 100 | 100 | 100 | 100 | 100 | 100 | 100 | 100 | 100 |
| Day 5 | 98 | 96 | 97 | 99 | 89 | 94 | 94 | 97 | 92 |
| Day 7 | 89 | 89 | 95 | 95 | 90 | 91 | 92 | 95 | 89 |
| Day 26 | 94 | 103 | 97 | 96 | 100 | 103 | 104 | 102 | 105 |

| | | | | | | | | | |
|---|---|---|---|---|---|---|---|---|---|
| Each numerical value in table is % | | | | | | | | | |

**[Table 12]**

| cEV concentration (ng/mL) | 0 | 4.5 | 41 | 370 |
|---|---|---|---|---|
| Day 0 | 100 | 100 | 100 | 100 |
| Day 7 | 99 | 97 | 98 | 109 |
| Day 17 | 112 | 107 | 110 | 107 |
| Day 21 | 99 | 127 | 147 | 140 |
| Day 26 | 91 | 120 | 123 | 124 |

| | | | | |
|---|---|---|---|---|
| Each numerical value in table is % | | | | |

### [Measurement reproducibility of CD9(ECD)-ALP conjugate]

The secondary calibrators C0, C1, C2 and C3 of a CD9(ECD)-ALP conjugate were measured by HI-1000 using the above R1, R2, R4 and R5 reagents including the R1 reagent, count values were acquired, and protein concentrations (ng/mL) were calculated. The measurement was performed ten times for each sample, and the coefficient of variation (CV) was calculated. The results are shown in Table 13. From Table 13, it can be seen that the values of CV are low even when the calibrators using the CD9(ECD)-ALP conjugate are continuously measured.

**[Table 13]**

| Number of trials | C0 | C1 | C2 | C3 |
|---|---|---|---|---|
| 1 | 0 | 1.57 | 5.31 | 16.52 |
| 2 | 0 | 1.56 | 5.49 | 16.06 |
| 3 | 0 | 1.74 | 4.89 | 16.31 |
| 4 | 0 | 1.64 | 4.86 | 15.54 |
| 5 | 0 | 1.50 | 5.07 | 15.63 |
| 6 | 0 | 1.57 | 5.23 | 15.91 |
| 7 | 0 | 1.60 | 5.10 | 15.54 |
| 8 | 0 | 1.41 | 5.27 | 16.41 |
| 9 | 0 | 1.49 | 5.73 | 15.09 |
| 10 | 0 | 1.74 | 5.09 | 16.37 |
| av | 0 | 1.58 | 5.20 | 15.94 |
| sd | 0 | 0.1 | 0.25 | 0.45 |
| cv | 300% | 6% | 5% | 3% |

| | | | | |
|---|---|---|---|---|
| Units of measured values other than CV are pg/ml | | | | |

Secondary calibrators C0 to C5 of a CD9(ECD)-ALP conjugate which were dispensed into a plurality of aliquots and stored at -80°C were measured by HI-1000 using the above R1, R2, R4 and R5 reagents including the R1 reagent, and count values were acquired. Three samples to be measured were randomly selected from a plurality of aliquot samples and thawed. The results are shown in Table 14. From Table 14, it can be seen that the error between samples is small in the calibrators using the CD9(ECD)-ALP conjugate.

From these, it can be seen that the calibrator using the CD9(ECD)-ALP conjugate has high stability as a measurement sample, and thus is excellent as a calibrator.

**[Table 14]**

| Sample | C0 | C1 | C2 | C3 | C4 | C5 |
|---|---|---|---|---|---|---|
| 1 | 472 | 818 | 1565 | 3829 | 11413 | 36538 |
| 2 | 463 | 810 | 1583 | 4083 | 11980 | 36821 |
| 3 | 469 | 805 | 1518 | 3804 | 11418 | 35661 |
| av | 468 | 811 | 1556 | 3905 | 11604 | 36340 |
| sd | 4 | 5 | 27 | 126 | 266 | 494 |
| cv | 1% | 1% | 2% | 3% | 2% | 1% |

### Example 2

### [Measurement of extracellular vesicles containing ALP using CD9(FL)-ALP conjugate as calibrator]

A CD9(FL)-ALP conjugate was prepared, and a specimen sample was measured.

### [Preparation of calibrators containing CD9(FL)-ALP]

Recombinant Human CD9 protein (Tagged), ab 152262, Abcam plc. was used as a polypeptide including an amino acid sequence of a membrane protein. Hereinafter, this polypeptide is also referred to as "CD9(FL)". CD9(FL) was a recombinant protein composed of an entire region of CD9. The amino acid sequence of CD9(FL) was shown in SEQ ID NO: 2. As ALP, BIAP similar to that in Example 1 was used. CD9(FL) and BIAP were covalently bound via a linker to obtain calibrators containing a conjugate (CD9(FL)-ALP). Alkaline Phosphatase Labeling Kit-SH (DOJINDO LABORATORIES) was used for binding between CD9(FL) and BIAP.

The CD9(FL)-ALP conjugate was measured by HI-1000 using the above R1, R2, R4 and R5 reagents, and count values were acquired. The measurement was performed three times for each conjugate, and the average thereof was taken. As a control, the R1 solution containing no capture antibody was also treated and measured in the same manner.

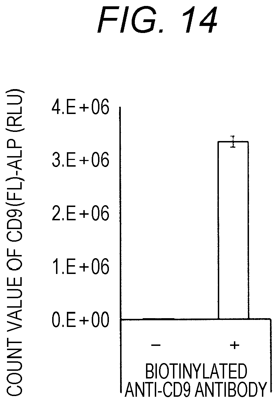

The measurement results are shown in Fig. 14. As shown in Fig. 14, it was found that an antibody-dependent signal can be acquired from the CD9(FL)-ALP conjugate. Therefore, it was shown that CD9(FL)-ALP can be used as a standard substance.

For the CD9(FL)-ALP conjugate, the absorbance (A280) of the diluted solution prepared using a diluent for calibrator was measured, and the protein concentration (ng/mL) was quantified. The results are shown in Table 15 below.

**[Table 15]**

| | C1 | C2 | C3 | C4 | C5 |
|---|---|---|---|---|---|
| CD9(FL)-ALP conjugate | 1.2 | 3.7 | 7.4 | 15 | 30 |

### (i) Preparation of calibration curve of protein concentration

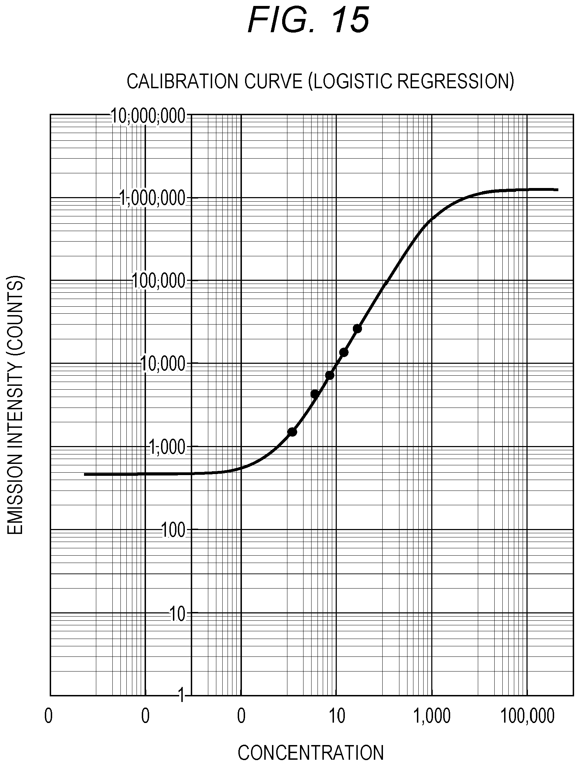

The calibrators C0 to C5 of a CD9(FL)-ALP conjugate in which the protein concentration was measured as described above were measured using the above-described R1, R2, R4 and R5 reagents and HI-1000, and count values were acquired for each calibrator. The measurement was performed three times for each sample. Based on the obtained count values, a calibration curve (calibration curve 3) was prepared based on logistic regression. The measurement results are shown in Table 16 and Fig. 15. From Table 16 and Fig. 15, it was shown that a calibration curve can be prepared using the calibrators C0 to C5 of a CD9(FL)-ALP conjugate.

**[Table 16]**

| | C0 | C1 | C2 | C3 | C4 | C5 |
|---|---|---|---|---|---|---|
| Protein concentration (ng/mL) | 0 | 1.0 | 3.7 | 7.4 | 15 | 30 |
| Count value | 471 | 1418 | 5355 | 7038 | 12971 | 24630 |
| | 444 | 1551 | 3679 | 7015 | 13793 | 26944 |
| | 474 | 1472 | 3805 | 7006 | 13855 | 26136 |
| av | 463 | 1480 | 4280 | 7020 | 13540 | 25903 |
| sd | 13 | 55 | 762 | 13 | 403 | 959 |
| cv | 3% | 4% | 18% | 0% | 3% | 4% |

The protein concentrations obtained by applying the count values obtained in the above measurement to the calibration curve 3 are shown in Table 17 below. From Table 17, it is found that the values of the protein concentrations acquired by applying the count values to the calibration curve 3 are almost the same as the values of the protein concentrations shown in Table 6. From these, it was shown that the calibration curve can be prepared using the CD9(FL)-ALP conjugate, and this conjugate can be used as a calibrator.

**[Table 17]**

| | C0 | C1 | C2 | C3 | C4 | C5 |
|---|---|---|---|---|---|---|
| Protein concentration (ng/mL) | 0 | 1.07 | 5.45 | 7.33 | 13.98 | 27.23 |
| | 0 | 1.21 | 3.58 | 7.30 | 14.91 | 29.88 |
| | 0 | 1.13 | 3.72 | 7.29 | 14.98 | 28.95 |
| av | 0 | 1.14 | 4.25 | 7.31 | 14.62 | 28.69 |
| sd | 0 | 0 | 1 | 0 | 0 | 1 |
| cv | 73% | 5% | 20% | 0% | 3% | 4% |
| Regression rate | - | 92% | 115% | 99% | 99% | 97% |

### (ii) Preparation of calibration curve of ALP activity value

For the CD9(FL)-ALP conjugate, the ALP activity value was valued by biochemical ALP measurement, and a calibration curve was prepared in the same manner as described above. The ALP activity value was valued as follows.

Biochemical ALP measurement was performed on the calibrator C5 of a CD9(FL)-ALP conjugate. For the measurement, Hitachi automatic analyzer 7180 was used as a measuring device, Iatro (registered trademark) ALP-IF R1/R2 was used as a measurement reagent, and enzyme calibrator plus was used as a calibrator, in the same manner as the valuing of the ALP activity value for a CD9(ECD)-ALP conjugate. According to the obtained measurement results, the calibrator C5 was valued by the ALP activity value. Since the calibrators of C0 to C4 had an activity value less than or equal to the lower limit of the measurement range of the measurement reagent, the ALP activity values were valued by calculating based on the ALP activity value of C5 and the dilution ratio.

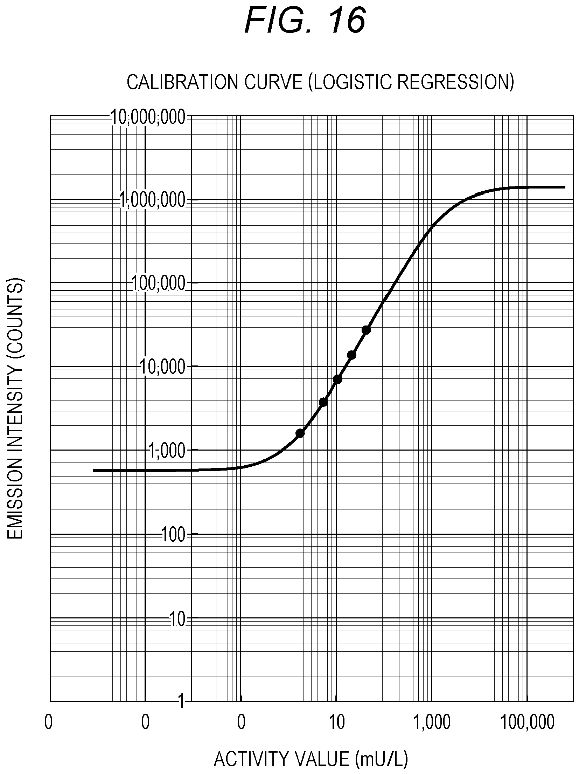

The calibrators C0 to C5 of a CD9(FL)-ALP conjugate valued by the ALP activity value were measured using the above-described R1, R2, R4 and R5 reagents and HI-1000, and count values were acquired for each calibrator. The measurement was performed three times for each sample. Based on the obtained count values, a calibration curve (calibration curve 4) was prepared based on logistic regression. The measurement results are shown in Table 18 and Fig. 16. From Table 18 and Fig. 16, it was shown that a calibration curve can be prepared using the calibrators C0 to C5 of a CD9(FL)-ALP conjugate in which the ALP activity value was valued.

**[Table 18]**

| | C0 | C1 | C2 | C3 | C4 | C5 |
|---|---|---|---|---|---|---|
| Activity value (mU/L) | 0 | 1.83 | 5.48 | 10.96 | 21.93 | 43.86 |
| Count value | 580 | 1579 | 3633 | 6334 | 13396 | 25803 |
| | 529 | 1585 | 3516 | 7086 | 13181 | 25645 |
| | 605 | 1463 | 3784 | 6808 | 13286 | 27774 |
| av | 571 | 1542 | 3644 | 6743 | 13288 | 26407 |
| sd | 32 | 56 | 110 | 310 | 88 | 969 |
| cv | 6% | 4% | 3% | 5% | 1% | 4% |

The ALP activity values obtained by applying the count values obtained in the above measurement to the calibration curve 4 is shown in Table 19 below. From Table 19, it is found that the ALP activity values acquired by applying the count values to the calibration curve 4 are almost the same as the ALP activity values shown in Table 18. From these, it was shown that the calibration curve can be prepared using the CD9(FL)-ALP conjugate, and this conjugate can be used as a calibrator.

**[Table 19]**

| | C0 | C1 | C2 | C3 | C4 | C5 |
|---|---|---|---|---|---|---|
| Activity value (mU/L) | 0.019 | 1.888 | 5.522 | 10.18 | 22.131 | 42.893 |
| | 0 | 1.899 | 5.318 | 11.465 | 21.769 | 42.629 |
| | 0.071 | 1.678 | 5.785 | 10.99 | 21.946 | 46.188 |
| av | 0.03 | 1.822 | 5.541 | 10.878 | 21.949 | 43.903 |
| sd | 0.03 | 0.102 | 0.191 | 0.530 | 0.148 | 1.619 |
| cv | 100% | 6% | 3% | 5% | 1% | 4% |
| Regression rate | - | 100% | 101% | 99% | 100% | 100% |

### (iii) Quantification of protein concentration using specimen sample

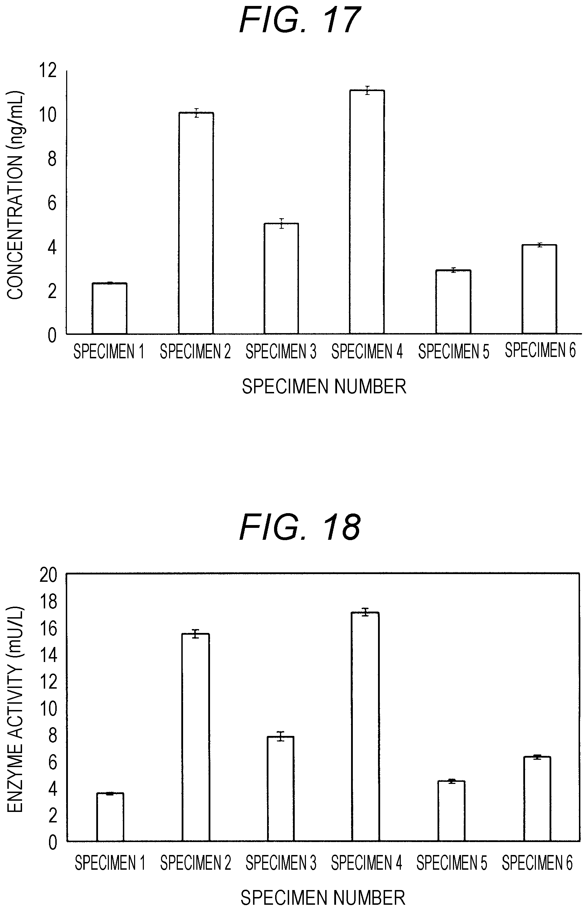

The protein concentrations of extracellular vesicles containing ALP were measured using the plasma specimen samples (6 specimens) of patients with history of cardiovascular disease. An experiment was performed on a sample obtained by thawing each frozen specimen sample and then diluting the sample 4-fold with a diluent for calibrator. Each prepared specimen sample was measured by HI-1000 using the above R1, R2, R4 and R5 reagents, and count values were acquired. The measurement was performed three times for each specimen sample. The protein concentrations of extracellular vesicles containing ALP contained in the specimen samples were measured by applying the obtained measured values to the calibration curve 3. The measurement results are shown in Fig. 17. From Fig. 17, it can be seen that the protein concentrations of extracellular vesicles containing ALP in the plasma specimens can be measured, and the obtained count values can be applied to the calibration curve. From this, it was shown that the protein concentrations of the specimen samples can be quantified using the calibration curve prepared using the CD9(FL)-ALP conjugate.

### (iv) Quantification of ALP activity value using specimen sample

The count values obtained in the above (iii) were applied to the calibration curve 4 to calculate ALP activity values of specimen samples. The results are shown in Fig. 18. From Fig. 18, it can be seen that the ALP activity values of extracellular vesicles containing ALP in the plasma specimens can be measured, and the obtained count values can be applied to the calibration curve. From this, it was shown that the ALP activity values of specimen samples can be quantified using the calibration curve prepared using the CD9(FL)-ALP conjugate.

### Example 3

### [Measurement of extracellular vesicles containing ALP using CD63(ECD)-ALP conjugate as calibrator]

A CD63(ECD)-ALP conjugate was prepared, and a specimen sample was measured.

### [Preparation of calibrators containing CD63(ECD)-ALP]

CD63 Protein, Human, Recombinant (ECD, His Tag), 11271-H08H, Sino Biological was used as a polypeptide including an amino acid sequence of a membrane protein. Hereinafter, this polypeptide is also referred to as "CD63(ECD)". CD63(ECD) was a recombinant protein composed of the extracellular domain of CD63. The amino acid sequence of CD63(ECD) was shown in SEQ ID NO: 3. Calibrators containing a conjugate (CD63(ECD)-ALP) were obtained in the same manner as in [Preparation of calibrators containing CD9(FL)-ALP] except for using CD63(ECD).

The CD63(ECD)-ALP conjugate was measured by HI-1000 using R1, R2, R4 and R5 reagents including the R1 reagent prepared using an anti-CD63 antibody (Clone: H5C6) instead of the anti-CD9 antibody, and count values were acquired. The measurement was performed three times for each conjugate, and the average thereof was taken. As a control, the R1 solution containing no capture antibody was also treated and measured in the same manner.

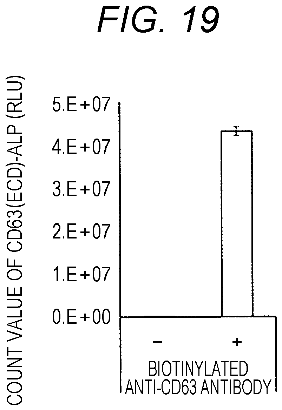

The measurement results are shown in Fig. 19. As shown in Fig. 19, it was found that an antibody-dependent signal can be acquired from the CD63(ECD)-ALP conjugate. Therefore, it was shown that the CD63(FL)-ALP conjugate can also be used as a standard substance.

For the CD63(ECD)-ALP conjugate, the absorbance (A280) of the diluted solution prepared using a diluent for calibrator was measured, and the protein concentration (ng/mL) was quantified. The results are shown in Table 20 below.

**[Table 20]**

| | C1 | C2 | C3 | C4 | C5 |
|---|---|---|---|---|---|
| CD63(ECD)-ALP conjugate | 2.5 | 12.5 | 62.5 | 313 | 1563 |

### (i) Preparation of calibration curve of protein concentration

For the calibrators C0 to C5 of a CD63(ECD)-ALP conjugate in which the protein concentration was measured as described above, ALP activity was measured using HI-1000 using the above-described R1, R2, R4 and R5 reagents, and count values were acquired for each calibrator. The measurement was performed three times for each sample. Based on the obtained count values, a calibration curve (calibration curve 5) was prepared based on logistic regression. The measurement results are shown in Table 21 and Fig. 20. From Table 21 and Fig. 20, it was shown that a calibration curve can be prepared using the calibrators C0 to C5 of a CD63(ECD)-ALP conjugate in which the protein concentration was valued.

**[Table 21]**

| | C0 | C1 | C2 | C3 | C4 | C5 |
|---|---|---|---|---|---|---|
| Protein concentration (ng/mL) | 0 | 2.5 | 12.5 | 62.5 | 313 | 1563 |
| Count | 427 | 770 | 2165 | 8693 | 41716 | 217822 |
| | 425 | 751 | 2125 | 9246 | 41308 | 204652 |
| | 416 | 743 | 2258 | 8490 | 42785 | 212680 |
| av | 422.7 | 754.7 | 2182.7 | 8809.7 | 41936.3 | 211718 |
| sd | 4.8 | 11.3 | 55.7 | 319.5 | 622.8 | 5419.5 |
| cv | 1% | 2% | 3% | 4% | 1% | 3% |

The protein concentrations obtained by applying the count values obtained in the above measurement to the calibration curve 5 are shown in Table 22 below. From Table 22, it is found that the values of the protein concentrations calculated by applying the count values to the calibration curve 5 are almost the same as the values of the protein concentrations shown in Table 21. From these, it was shown that the calibration curve can be prepared using the CD63(ECD)-ALP conjugate, and this conjugate can be used as a calibrator.

**[Table 22]**

| | C0 | C1 | C2 | C3 | C4 | C5 |
|---|---|---|---|---|---|---|
| Protein concentration (ng/mL) | 0.032 | 2.559 | 12.865 | 61.193 | 306.202 | 1615.937 |
| | 0.017 | 2.419 | 12.569 | 65.291 | 303.172 | 1517.898 |
| | 0 | 2.360 | 13.553 | 59.689 | 314.14 | 1577.658 |
| av | 0.02 | 2.45 | 13 | 62.06 | 307.84 | 1570.5 |
| sd | 0.013 | 0.084 | 0.412 | 2.367 | 4.625 | 40.343 |
| cv | 80% | 3% | 3% | 4% | 2% | 3% |
| Regression rate | - | 98% | 104% | 99% | 99% | 101% |

### (ii) Preparation of calibration curve of ALP activity value

For the CD63(ECD)-ALP conjugate, the ALP activity value was valued by biochemical ALP measurement, and a calibration curve was prepared in the same manner as described above. The ALP activity value was valued in the same manner as the valuing of the ALP activity value for the CD9(FL)-ALP conjugate.

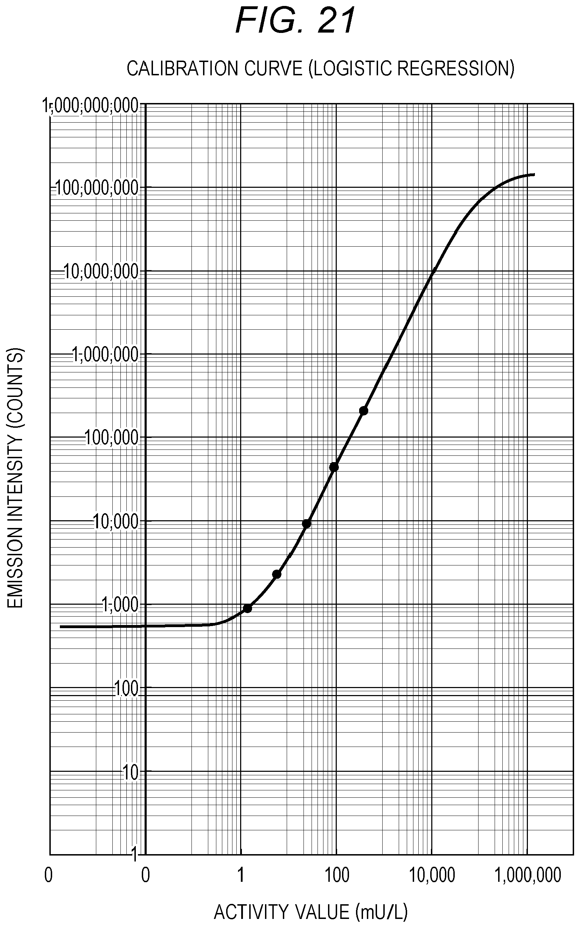

The calibrators C0 to C5 of a CD63(ECD)-ALP conjugate valued by the ALP activity value were measured using the above-described R1, R2, R4 and R5 reagents and HI-1000, and count values were acquired for each calibrator. The measurement was performed three times for each sample. Based on the obtained count values, a calibration curve (calibration curve 6) was prepared based on logistic regression. The measurement results are shown in Table 23 and Fig. 21. From Table 23 and Fig. 21, it was shown that a calibration curve can be prepared using the calibrators C0 to C5 of a CD63(ECD)-ALP conjugate in which the ALP activity value was valued.

**[Table 23]**

| | C0 | C1 | C2 | C3 | C4 | C5 |
|---|---|---|---|---|---|---|
| Activity value (mU/L) | 0 | 1.43 | 5.71 | 22.84 | 91.36 | 365.43 |
| Count value | 526 | 900 | 2255 | 9143 | 42991 | 206229 |
| | 533 | 871 | 2383 | 9245 | 42229 | 212064 |
| | 568 | 916 | 2243 | 9076 | 42668 | 207075 |
| av | 542 | 896 | 2294 | 9155 | 42629 | 208456 |
| sd | 18 | 19 | 63 | 69 | 312 | 2575 |
| cv | 3% | 2% | 3% | 1% | 1% | 1% |

The ALP activity values obtained by applying the count values obtained in the above measurement to the calibration curve 6 is shown in Table 24 below. From Table 24, it is found that the ALP activity values calculated by applying the count values to the calibration curve 6 are almost the same as the ALP activity values shown in Table 23. From these, it was shown that the calibration curve can be prepared using the CD63(ECD)-ALP conjugate, and this conjugate can be used as a calibrator.

**[Table 24]**

| | C0 | C1 | C2 | C3 | C4 | C5 |
|---|---|---|---|---|---|---|
| Activity value (mU/L) | 0 | 1.439 | 5.618 | 22.87 | 91.714 | 362.237 |
| | 0 | 1.337 | 5.982 | 23.106 | 90.28 | 371.172 |
| | 0.145 | 1.494 | 5.584 | 22.715 | 91.106 | 363.534 |
| av | 0.048 | 1.423 | 5.728 | 22.897 | 91.033 | 365.648 |
| sd | 0.069 | 0.065 | 0.18 | 0.161 | 0.588 | 3.942 |
| cv | 141% | 5% | 3% | 1% | 1% | 1% |
| Regression rate | - | 100% | 100% | 100% | 100% | 100% |

### (iii) Quantification of protein concentration using specimen sample

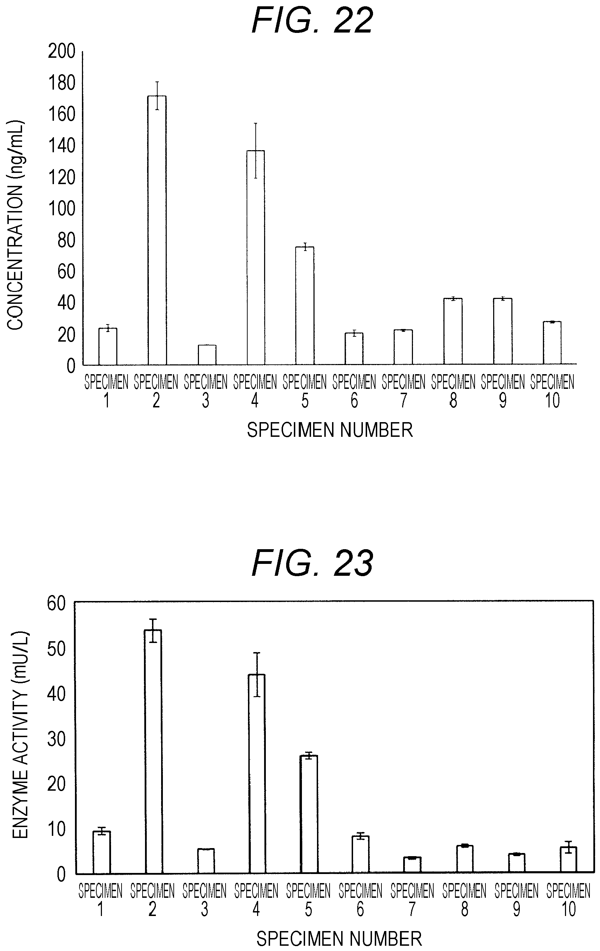

The protein concentrations of extracellular vesicles containing ALP were measured using plasma specimen samples (10 specimens) obtained from patients with end-stage renal failure. Calcification of blood vessels is often observed in the patients with end-stage renal failure. An experiment was performed on a sample obtained by thawing each frozen specimen sample and then diluting the sample 4-fold with a diluent for calibrator. Each prepared sample was measured by HI-1000 using the above R1, R2, R4 and R5 reagents, and count values were acquired. The measurement was performed three times for each specimen sample. The protein concentrations of extracellular vesicles containing ALP contained in the specimen samples were measured by applying the obtained measured values to the calibration curve 5. The measurement results are shown in Fig. 22. From Fig. 22, it can be seen that the protein concentrations of extracellular vesicles containing ALP in the plasma specimen samples can be measured, and the obtained count values can be applied to the calibration curve. From this, it was shown that the protein concentrations of the specimen samples can be quantified using the calibration curve prepared using the CD63(ECD)-ALP conjugate.

### (iv) Quantification of ALP activity value using specimen sample

The count values obtained in the above (iii) were applied to the calibration curve 6 to calculate ALP activity values of specimen samples. The results are shown in Fig. 23. From Fig. 23, it can be seen that the ALP activity values of extracellular vesicles containing ALP in the plasma specimens can be measured, and the obtained count values can be applied to the calibration curve. From this, it was shown that the ALP activity values of specimen samples can be quantified using the calibration curve prepared using the CD63(ECD)-ALP conjugate.

### Example 4

### [Measurement of extracellular vesicles containing ALP using CD81(ECD)-ALP conjugate as calibrator]

A CD81(ECD)-ALP conjugate was prepared, and a specimen sample was measured.

### [Preparation of calibrators containing CD81(ECD)-ALP]

CD81 Protein, Human, Recombinant (ECD, His Tag), 14244-H07H, Sino Biological was used as a polypeptide including an amino acid sequence of a membrane protein. Hereinafter, this polypeptide is also referred to as "CD81(ECD)". CD81(ECD) was a recombinant protein composed of the extracellular domain of CD81. The amino acid sequence of CD81(ECD) was shown in SEQ ID NO: 4. Calibrators containing a conjugate (CD81(ECD)-ALP) were obtained in the same manner as in [Preparation of calibrators containing CD9(FL)-ALP] except for using CD81(ECD).

The CD81(ECD)-ALP conjugate was measured by HI-1000 using R1, R2, R4 and R5 reagents including the R1 reagent prepared using an anti-CD81 antibody (Clone: 5A6) instead of the anti-CD9 antibody, and count values were acquired. The measurement was performed three times for each conjugate, and the average thereof was taken. As a control, the R1 solution containing no capture antibody was also treated and measured in the same manner.

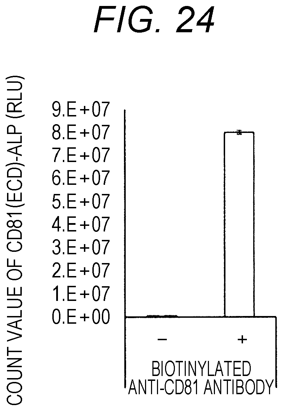

The measurement results are shown in Fig. 24. As shown in Fig. 24, it is found that ALP activity is obtained from the CD81(ECD)-ALP conjugate. From this, it was shown that the CD81(ECD)-ALP conjugate can also be used as a standard substance.

For the CD81(ECD)-ALP conjugate, the absorbance (A280) of the diluted solution prepared using a diluent for calibrator was measured, and the protein concentration (ng/mL) was quantified. The results are shown in Table 25 below.

**[Table 25]**

| | C1 | C2 | C3 | C4 | C5 |
|---|---|---|---|---|---|
| CD81(ECD)-ALP conjugate | 4 | 20 | 100 | 500 | 2500 |

### (i) Preparation of calibration curve of protein concentration

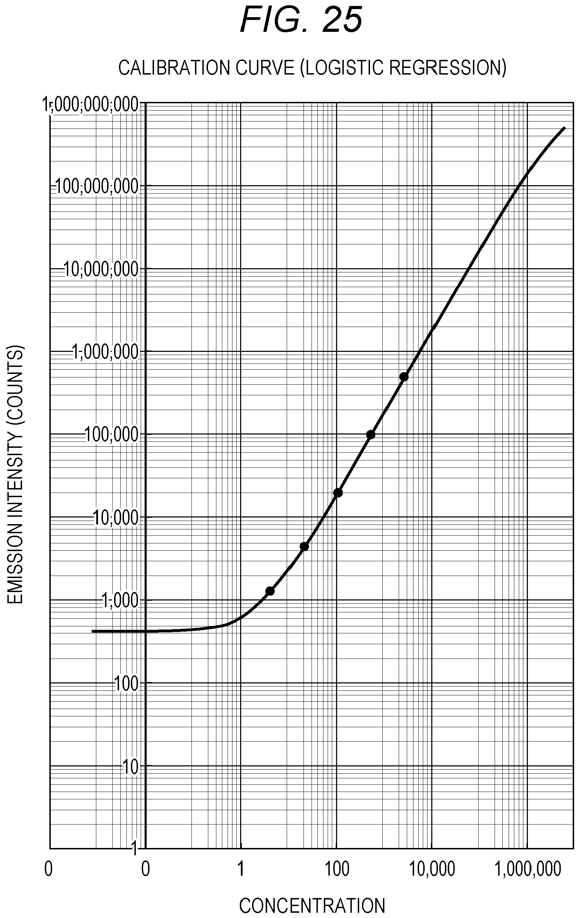

For the calibrators C0 to C5 of a CD81(ECD)-ALP conjugate in which the protein concentration was measured as described above, ALP activity was measured using HI-1000 using the above-described R1, R2, R4 and R5 reagents, and count values were acquired for each calibrator. The measurement was performed three times for each sample. Based on the obtained count values, a calibration curve (calibration curve 7) was prepared based on logistic regression. The measurement results are shown in Table 26 and Fig. 25. From Table 26 and Fig. 25, it was shown that a calibration curve can be prepared using the calibrators C0 to C5 of a CD81(ECD)-ALP conjugate.

**[Table 26]**

| | C0 | C1 | C2 | C3 | C4 | C5 |
|---|---|---|---|---|---|---|
| Protein concentration (ng/mL) | 0 | 4 | 20 | 100 | 500 | 2500 |
| Count value | 445 | 1271 | 4478 | 19953 | 101336 | 498481 |
| | 410 | 1304 | 4383 | 20025 | 100077 | 496133 |
| | 427 | 1277 | 4635 | 19626 | 99595 | 486280 |
| av | 427.33 | 1284 | 4498.67 | 19868 | 100336 | 493631.33 |
| sd | 14.29 | 14.35 | 103.91 | 173.63 | 733.98 | 5285.82 |
| cv | 3% | 1% | 2% | 1% | 1% | 1% |

The protein concentrations obtained by applying the count values obtained in the above measurement to the calibration curve 7 are shown in Table 27 below. From Table 27, it is found that the values of the protein concentrations calculated by applying the count values to the calibration curve 7 are almost the same as the values of the protein concentrations shown in Table 26. From these, it was shown that the calibration curve can be prepared using the CD81(ECD)-ALP conjugate, and this conjugate can be used as a calibrator.

**[Table 27]**

| | C0 | C1 | C2 | C3 | C4 | C5 |
|---|---|---|---|---|---|---|
| Protein concentration (ng/mL) | 0.081 | 4.033 | 19.712 | 96.743 | 509.481 | 2561.925 |
| | 0 | 4.192 | 19.245 | 97.104 | 503.052 | 2549.703 |
| | 0 | 4.062 | 20.485 | 95.105 | 500.59 | 2498.426 |
| av | 0.027 | 4.096 | 19.814 | 96.318 | 504.374 | 2536.685 |
| sd | 0.038 | 0.069 | 0.512 | 0.87 | 3.748 | 27.509 |
| cv | 141% | 2% | 3% | 1% | 1% | 1% |
| Regression rate | - | 102% | 99% | 96% | 101% | 101% |

### (ii) Preparation of calibration curve of ALP activity value

For the CD81(ECD)-ALP conjugate, the ALP activity value was valued by biochemical ALP measurement, and a calibration curve was prepared in the same manner as described above. The ALP activity value was valued in the same manner as the valuing of the ALP activity value for the CD9(FL)-ALP conjugate.

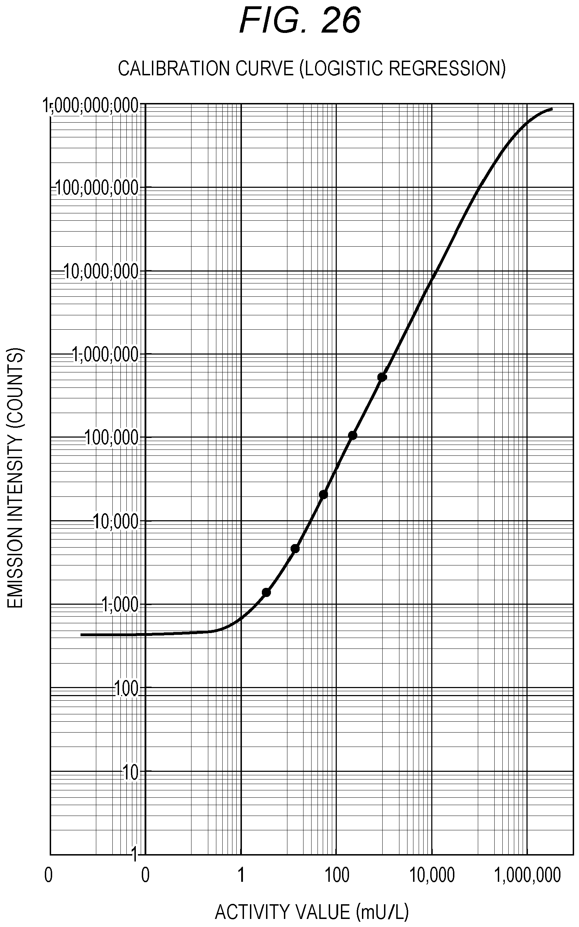

The calibrators C0 to C5 of a CD81(ECD)-ALP conjugate valued by the ALP activity value were measured using the above-described R1, R2, R4 and R5 reagents and HI-1000, and count values were acquired for each calibrator. The measurement was performed three times for each sample. Based on the obtained count values, a calibration curve (calibration curve 8) was prepared based on logistic regression. The measurement results are shown in Table 28 and Fig. 26. From Table 28 and Fig. 26, it was shown that a calibration curve can be prepared using the calibrators C0 to C5 of a CD81(ECD)-ALP conjugate in which the ALP activity value was valued.

**[Table 28]**

| | C0 | C1 | C2 | C3 | C4 | C5 |
|---|---|---|---|---|---|---|
| Activity value (mU/L) | 0 | 3.45 | 13.80 | 55.20 | 220.80 | 883.16 |
| Count value | 445 | 1378 | 4652 | 20935 | 104688 | 503320 |
| | 410 | 1427 | 4597 | 20359 | 99518 | 496285 |
| | 427 | 1343 | 4611 | 20894 | 101942 | 493998 |
| av | 427 | 1383 | 4620 | 20729 | 102049 | 497868 |
| sd | 14 | 34 | 23 | 262 | 2112 | 3967 |
| cv | 3% | 2% | 1% | 1% | 2% | 1% |

The ALP activity values obtained by applying the count values obtained in the above measurement to the calibration curve 8 is shown in Table 29 below. From Table 29, it is found that the ALP activity values calculated by applying the count values to the calibration curve 8 are almost the same as the ALP activity values shown in Table 28. From these, it was shown that the calibration curve can be prepared using the CD81(ECD)-ALP conjugate, and this conjugate can be used as a calibrator.

**[Table 29]**

| | C0 | C1 | C2 | C3 | C4 | C5 |
|---|---|---|---|---|---|---|
| Activity value (mU/L) | 0.106 | 3.584 | 13.379 | 53.987 | 226.943 | 910.934 |
| | 0 | 3.747 | 13.225 | 52.646 | 216.975 | 899.667 |
| | 0 | 3.468 | 13.264 | 53.891 | 221.656 | 896 |
| av | 0.035 | 3.6 | 13.289 | 53.508 | 221.858 | 902.2 |
| sd | 0.05 | 0.115 | 0.065 | 0.611 | 4.072 | 6.355 |
| cv | 141% | 3% | 0% | 1% | 2% | 1% |
| Regression rate | - | 104% | 96% | 97% | 100% | 102% |

### (iii) Quantification of protein concentration using specimen sample

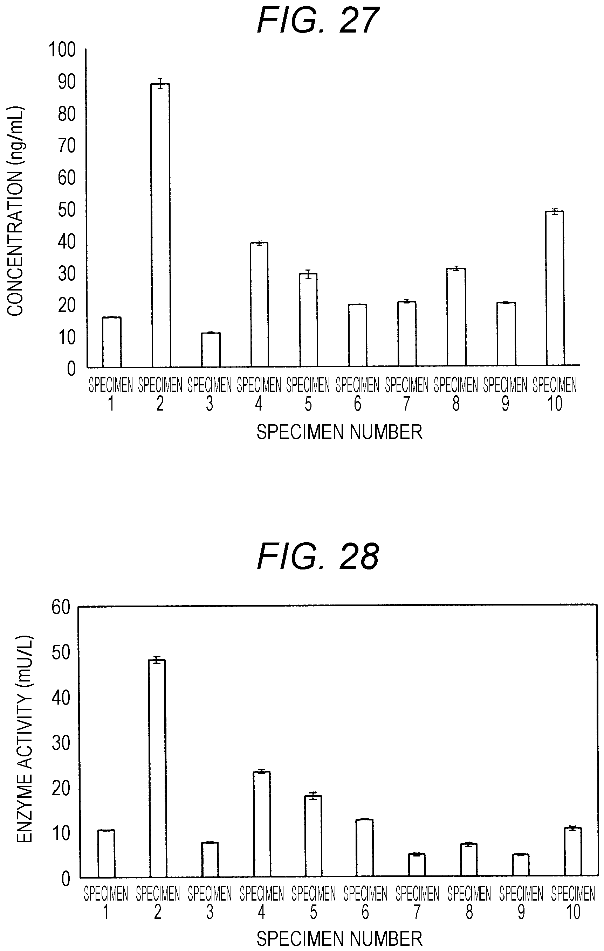

The protein concentrations of extracellular vesicles containing ALP were measured using plasma specimens (10 specimens) obtained from patients with end-stage renal failure. An experiment was performed on a sample obtained by thawing each frozen specimen sample and then diluting the sample 4-fold with a diluent for calibrator. Each prepared specimen sample was measured by HI-1000 using the above R1, R2, R4 and R5 reagents, and count values were acquired. The measurement was performed three times for each specimen sample. The protein concentrations of extracellular vesicles containing ALP contained in the specimen samples were measured by applying the obtained measured values to the calibration curve 7. The measurement results are shown in Fig. 27. From Fig. 27, it can be seen that the protein concentrations of extracellular vesicles containing ALP in the plasma specimen samples can be measured, and the obtained count values can be applied to the calibration curve. From this, it was shown that the protein concentrations of the specimen samples can be quantified using the calibration curve prepared using the CD81(ECD)-ALP conjugate.

### (iv) Quantification of ALP activity value using specimen sample

The count values obtained in the above (iii) were applied to the calibration curve 8 to calculate ALP activity values of specimen samples. The results are shown in Fig. 28. From Fig. 28, it can be seen that the ALP activity values of extracellular vesicles containing ALP in the plasma specimens can be measured, and the obtained count values can be applied to the calibration curve. From this, it was shown that the ALP activity values of specimen samples can be quantified using the calibration curve prepared using the CD81(ECD)-ALP conjugate.

### Example 5

### [Preparation of calibrators containing Annexin I(FL)-ALP]

Annexin I/ANXA1 Protein, Human, Recombinant (His Tag), 3770-AN-050, R&D Systems, Inc. was used as a polypeptide including an amino acid sequence of a membrane protein. Hereinafter, this polypeptide is also referred to as "Annexin I(FL)". Annexin I(FL) was a recombinant protein composed of an entire region of Annexin I. The amino acid sequence of Annexin I(FL) was shown in SEQ ID NO: 5. Calibrators containing a conjugate (Annexin I(FL)-ALP) were obtained in the same manner as in [Preparation of calibrators containing CD9(FL)-ALP] except for using Annexin I(FL).

The Annexin I(FL)-ALP conjugate was measured by HI-1000 using R1, R2, R4 and R5 reagents including the R1 reagent prepared using an anti-Annexin I antibody (Clone: EH17a) instead of the anti-CD9 antibody, and count values were acquired. The measurement was performed three times for each conjugate, and the average thereof was taken. As a control, the R1 solution containing no capture antibody was also treated and measured in the same manner.

The measurement results are shown in Fig. 29. As shown in Fig. 29, it is found that ALP activity is obtained from the Annexin I(FL)-ALP conjugate. From this, it was shown that the Annexin I(FL)-ALP conjugate can also be used as a standard substance.

### Example 6

### [Preparation of calibrators containing Annexin VI(FL)-ALP]

Annexin VI/ANXA6 Protein, Human, Recombinant (His Tag), 11161-H08E, Sino Biological Co., Ltd. was used as a polypeptide including an amino acid sequence of a membrane protein. Hereinafter, this polypeptide is also referred to as "Annexin VI(FL)". Annexin VI(FL) was a recombinant protein composed of an entire region of Annexin VI. The amino acid sequence of Annexin VI(FL) was shown in SEQ ID NO: 6. Calibrators containing a conjugate (Annexin VI(FL)-ALP) were obtained in the same manner as in [Preparation of calibrators containing CD9(FL)-ALP] except for using Annexin VI(FL).

The Annexin VI(FL)-ALP conjugate was measured by HI-1000 using R1, R2, R4 and R5 reagents including the R1 reagent prepared using an anti-Annexin VI antibody (Clone: E-5) instead of the anti-CD9 antibody, and count values were acquired. The measurement was performed three times for each conjugate, and the average thereof was taken. As a control, the R1 solution containing no capture antibody was also treated and measured in the same manner.

The measurement results are shown in Fig. 30. As shown in Fig. 30, it is found that ALP activity is obtained from the Annexin VI(FL)-ALP conjugate. From this, it was shown that the Annexin VI(FL)-ALP conjugate can also be used as a standard substance.

## Claims

1. A method for measuring an activity of alkaline phosphatase contained in an extracellular vesicle in a sample, comprising
acquiring an activity value of alkaline phosphatase contained in an extracellular vesicle in a sample using a calibrator,
wherein the calibrator comprises a conjugate in which a polypeptide having alkaline phosphatase activity and a polypeptide comprising an amino acid sequence of a membrane protein present on a surface of the extracellular vesicle are covalently bound.

2. The method according to claim 1, comprising:
a first immobilization step of immobilizing the extracellular vesicle contained in the sample on a first solid phase;
a first measurement step of measuring a signal generated by contacting the immobilized extracellular vesicle with a substrate of alkaline phosphatase;
a second immobilization step of immobilizing the conjugate contained in the calibrator on a second solid phase;
a second measurement step of measuring a signal generated by contacting the immobilized conjugate with a substrate of alkaline phosphatase; and
acquiring the activity value of alkaline phosphatase contained in the extracellular vesicle in the sample from a measured value obtained in the first measurement step and a measured value obtained in the second measurement step.

3. The method according to claim 2, wherein the first immobilization step comprises contacting the sample, the first solid phase, and a capture antibody capable of binding to the membrane protein contained in the extracellular vesicle in the sample with each other to form an immune complex of the extracellular vesicle and the capture antibody on the first solid phase.

4. The method according to claim 2 or 3, wherein the second immobilization step comprises contacting the calibrator, the second solid phase, and a capture antibody capable of binding to the membrane protein contained in the conjugate with each other to form an immune complex of the conjugate and the capture antibody on the second solid phase.

5. The method according to any of claims 1 to 4, wherein the amino acid sequence of the membrane protein comprises an amino acid sequence selected from the group consisting of an extracellular domain of CD9, an extracellular domain of CD63, an extracellular domain of CD81, Annexin I, Annexin VI, and Pit 1.

6. The method according to any of claims 1 to 5, wherein the alkaline phosphatase is one selected from the group consisting of tissue non-specific alkaline phosphatase, small intestine alkaline phosphatase, placenta alkaline phosphatase, and germ cell alkaline phosphatase.

7. The method according to claim 3, wherein the capture antibody in the first immobilization step is at least one selected from the group consisting of an anti-CD9 antibody, an anti-CD63 antibody, an anti-CD81 antibody, an anti-Annexin I antibody, an anti-Annexin VI antibody, and an anti-Pit 1 antibody.

8. The method according to claim 4, the capture antibody in the second immobilization step is at least one selected from the group consisting of an anti-CD9 antibody, an anti-CD63 antibody, an anti-CD81 antibody, an anti-Annexin I antibody, an anti-Annexin VI antibody, and an anti-Pit 1 antibody.

9. The method according to any of claims 1 to 8, wherein the polypeptide having alkaline phosphatase activity is a recombinant polypeptide, and/or the polypeptide comprising an amino acid sequence of the membrane protein is a recombinant polypeptide.

10. The method according to any of claims 1 to 9, wherein the sample is a blood sample.

11. The method according to any of claims 1 to 10, wherein the calibrator is a reagent containing the conjugate.

12. The method according to any of claims 1 to 11, wherein the calibrator is a reagent set comprising a plurality of reagents each comprising the conjugate, and concentrations of the conjugate in the plurality of reagents are different from each other.

13. The method according to claim 2,
wherein the calibrator is a reagent set comprising a plurality of reagents each comprising the conjugate, and concentrations of the conjugate in the plurality of reagents are different from each other, and
wherein the second measurement step is performed for each of conjugates derived from a plurality of reagents having different concentrations, and acquiring the activity value of alkaline phosphatase contained in the extracellular vesicle in the sample is performed based on a calibration curve prepared from measured values obtained from the conjugates derived from a plurality of reagents having different concentrations.

14. A calibrator suitable for measuring activity of alkaline phosphatase contained in an extracellular vesicle in a sample, **characterized in that**
the calibrator comprises a conjugate in which a polypeptide having alkaline phosphatase activity and a polypeptide including an amino acid sequence of a membrane protein present on a surface of an extracellular vesicle are covalently bound.

15. The use of a conjugate as a calibrator for measuring activity of alkaline phosphatase contained in an extracellular vesicle in a sample, wherein in said conjugate a polypeptide having alkaline phosphatase activity and a polypeptide including an amino acid sequence of a membrane protein present on a surface of an extracellular vesicle are covalently bound.
